(19) Europäisches Patentamt
European Patent Office
Office européen des brevets

(11) EP 4 810 523 A1

(12) **EUROPEAN PATENT APPLICATION**
published in accordance with Art. 153(4) EPC

(43) Date of publication:
**23.09.2026 Bulletin 2026/39**

(21) Application number: **24890673.7**

(22) Date of filing: **12.11.2024**

(51) International Patent Classification (IPC):
***C07K 19/00** (2006.01)* ***C12N 15/62** (2006.01)*

(52) Cooperative Patent Classification (CPC):
**C07K 19/00; C12N 15/62**

(86) International application number:
**PCT/CN2024/131610**

(87) International publication number:
**WO 2025/103318 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.11.2023 CN 202311515785**

(71) Applicant: **Shenyang Sunshine Pharmaceutical Co., Ltd.**
**Shenyang, Liaoning 110027 (CN)**

(72) Inventors:
- **LOU, Jing**
  **Shenyang, Liaoning 110027 (CN)**
- **ZHANG, Xuesai**
  **Shenyang, Liaoning 110027 (CN)**
- **HUANG, Haomin**
  **Shenyang, Liaoning 110027 (CN)**

(74) Representative: **dompatent Partnerschaft von Patentanwälten und Rechtsanwälten mbB**
**Deichmannhaus am Dom**
**Bahnhofsvorplatz 1**
**50667 Köln (DE)**

Remarks:
The complete document including Reference Table(s) and the Sequence Listing(s) can be downloaded from the EPO website

# (54) C3B-TARGETING BIFUNCTIONAL FUSION PROTEIN, PREPARATION METHOD THEREFOR AND USE THEREOF

(57) A C3b-targeting bifunctional fusion protein, a preparation method therefor and the use thereof. The C3b-targeting bifunctional fusion protein comprises a CRIg domain variant, an FH domain and a linker. The fusion protein has high affinity for human complement C3b, can effectively inhibit the activation of a plurality of complement pathways, can effectively inhibit the hemolytic reaction of complement pathway activation, and exhibits high affinity to FcRn.



Processed by Luminess, 75001 PARIS (FR)

## Description

### TECHNICAL FIELD

The present invention relates to the field of fusion proteins, specifically, to a C3b-targeting bifunctional fusion protein, preparation method therefor, and use thereof.

### BACKGROUND ART

The complement system consists of more than 30 proteins widely present in serum, tissue fluid, and on cell membrane surfaces, including complement activation factors, inhibitory factors, and complement receptors; it participates in specific and non-specific immunity of the body, manifesting as pro-inflammatory effects, opsonophagocytic effects, and cytolytic effects. The complement system can generally be activated through three pathways: the classical pathway (CP) can be activated by IgG and IgM antibodies; the lectin pathway (LP) can be activated by mannose on bacterial surfaces; while the alternative pathway (AP) can be activated by various components such as pathogen cell wall/membrane components or C3b analogs like cobra venom factor. C3 is the central molecule of the complement system and the convergence point of the three activation pathways of the complement system; C3b, generated by hydrolysis of C3 by C3 convertase, is a core component of C3 convertase (C3bBb) and C5 convertase (C3bBbC3b, C4bC2bC3b), and is a key protein affecting the activation of the mid-cascade reaction and the terminal pathway of the complement pathway. During complement activation, to prevent damage to the body, multiple regulatory proteins are involved, including Factor H (FH), Factor I (FI), and Factor B (FB) in the circulation. FH not only promotes the rate of FI-mediated inactivation of C3b but also competitively inhibits the binding of FB to C3b, and can also dissociate C3b from C3bBb, thereby accelerating the inactivation of C3bBb and inhibiting complement pathway activation; the C3b receptor CRIg expressed on cell membrane surfaces, because it specifically binds to the β chain of C3b, thereby occupying the binding site for substrates, thereby inhibiting the activity of C3 convertase and C5 convertase, exerting an inhibitory function on complement pathway activation.

The complement system is an important part of the innate immune system. Imbalance of complement is crucial in the pathogenesis of various diseases, including rare and common diseases, with broad impact, covering from acute inflammations such as ocular diseases and periodontal diseases to chronic diseases such as cancer, autoimmune diseases, neurodegenerative diseases, kidney diseases, and chronic hemolytic diseases. Currently approved complement drugs are mainly used to treat rare diseases such as paroxysmal nocturnal hemoglobinuria (PNH), atypical hemolytic uremic syndrome (aHUS), generalized myasthenia gravis (gMG), neuromyelitis optica (NMO), and are gradually expanding to non-rare diseases such as age-related macular degeneration (AMD) and geographic atrophy (GA).

Given that currently marketed inhibitors targeting C3 and/or C5 proteins have certain side effects and their therapeutic effects remain unsatisfactory, the present invention intends to develop a C3b-targeting bifunctional fusion protein based on the natural inhibitory proteins of C3b, FH and CRIg, for the treatment of diseases associated with abnormal activation of the complement system, expecting that the obtained CRIg-FH fusion protein will have lower side effects and better therapeutic effects compared to currently marketed inhibitors targeting C3 and/or C5 proteins.

### SUMMARY OF THE INVENTION

An object of the present invention is to provide a sequence-engineered CRIg-FH fusion protein or CRIg-FH-Fc fusion protein.

The fusion protein of the present invention has significantly improved affinity and complement inhibitory activity. A further object of the present invention is to provide a nucleic acid molecule encoding said fusion protein; to provide an expression vector comprising said nucleic acid molecule; to provide a host cell comprising said expression vector; to provide a method for preparing said fusion protein; to provide a pharmaceutical composition comprising said fusion protein; to provide a use of said fusion protein or said pharmaceutical composition in the manufacture of a medicament for preventing or treating a disease associated with complement activation; to provide a method of using said fusion protein or said pharmaceutical composition for preventing or treating a disease associated with complement activation.

In a first aspect of the present invention, there is provided a C3b-targeting bifunctional fusion protein, comprising:

a) a CRIg domain variant, wherein the variant comprises a mutation selected from the group consisting of: M86Y; or a combination of Q64R and M86Y, relative to SEQ ID NO: 2; and
b) an FH domain.

In another preferred embodiment, the C-terminus of said CRIg extracellular domain is directly or indirectly linked to the N-terminus of the FH domain, or the N-terminus of the CRIg extracellular domain is directly or indirectly linked to the

C-terminus of the FH domain.

In another preferred embodiment, the FH domain comprises the amino acid sequence as shown in SEQ ID NO: 4.

In another preferred embodiment, the C3b-targeting bifunctional fusion protein further comprises:

c) a linker connecting a) and b).

In another preferred embodiment, in the C3b-targeting bifunctional fusion protein, the linkage order from N-terminus to C-terminus is: CRIg domain variant - linker - FH domain. In another preferred embodiment, the C-terminus of the FH domain is directly or indirectly linked to the N-terminus of the Fc domain variant, or the C-terminus of the CRIg extracellular domain is directly or indirectly linked to the N-terminus of the Fc domain variant.

In another preferred embodiment, the linker is a flexible linker, preferably (GS4)n or (SG4)m, wherein n or m is a positive integer selected from 1, 2, 3, 4, 5.

In another preferred embodiment, the C3b-targeting bifunctional fusion protein further comprises:

d) an Fc domain variant.

In another preferred embodiment, the Fc domain variant comprises an Fc domain variant selected from the group consisting of IgG1-Fc, IgG2-Fc, IgG3-Fc region or IgG4-Fc.

In another preferred embodiment, the Fc domain variant is IgG4-Fc; preferably, it comprises an amino acid mutation S228P at position 228 in the human IgG4 constant region Fc sequence, and/or comprises a mutation selected from the group consisting of: M252Y/S254T/T256E.

In another preferred embodiment, the bifunctional fusion protein retains the biological activity of the elements a), b) and/or d).

In another preferred embodiment, the bifunctional fusion protein further has one or more of the following functions:

(a) activity of binding human complement C3b;
(b) inhibition of the complement alternative pathway;
(c) inhibition of the complement classical pathway;
(d) inhibition of the complement lectin pathway;
(e) inhibition of alternative pathway and classical pathway activation;
(e) inhibition of erythrocyte lysis.

In a preferred embodiment, the bifunctional fusion protein is a single-chain structure.

In another preferred embodiment, the bifunctional fusion protein is a dimer; preferably a homodimer or heterodimer.

In another preferred embodiment, the bifunctional fusion protein comprises a first polypeptide chain and a second polypeptide chain, wherein the first polypeptide chain comprises the CRIg domain variant, a first FH domain, and a first Fc domain variant; the second polypeptide chain comprises the CRIg domain variant, a second FH domain, and a second Fc domain variant,

wherein the first Fc domain variant and the second Fc domain variant are capable of interacting with each other to form a dimer.

In another preferred embodiment, the first polypeptide chain further comprises a first linker connecting the CRIg domain variant and the first FH domain;

the second polypeptide chain further comprises a second linker connecting the CRIg domain variant and the second FH domain.

In another preferred embodiment, the first FH domain and the second FH domain are identical or different.

In another preferred embodiment, the first Fc domain variant and the second Fc domain variant are identical or different.

In another preferred embodiment, the first polypeptide chain and the second polypeptide chain are identical.

In another preferred embodiment, the first polypeptide chain and/or the second polypeptide chain of the bifunctional fusion protein has a structure shown by Formula I or II:

X-L-Y-Z (Formula I)

Y-L-X-Z (Formula II)

wherein,

X is a CRIg domain variant, the variant comprising, relative to SEQ ID NO: 2, a mutation selected from the group consisting of: M86Y; or a combination of Q64R and M86Y;
Y is an FH domain;

Z is absent or an Fc domain variant;
L is absent or a linker;

- represents a peptide bond or peptide linker.

In another preferred embodiment, any two of the X, Y, Z are connected in a head-to-head, head-to-tail, or tail-to-tail manner.
In another preferred embodiment, the "head" refers to the N-terminus of a polypeptide or fragment thereof, especially the N-terminus of a wild-type polypeptide or fragment thereof.
In another preferred embodiment, the "tail" refers to the C-terminus of a polypeptide or fragment thereof, especially the C-terminus of a wild-type polypeptide or fragment thereof.
In another preferred embodiment, the sequence of the X is residues 1-119 of the sequence shown in SEQ ID NO. 9, or residues 1-119 of the sequence shown in SEQ ID NO. 10; or residues 1-119 of the sequence shown in SEQ ID NO. 11.
In another preferred embodiment, the sequence of the Y is as shown in SEQ ID NO: 4.
In another preferred embodiment, the nucleotide sequence encoding Y is as shown in SEQ ID NO: 3.
In another preferred embodiment, the sequence of the L is $(SG_4)_3$ or $(G_4S)_3$.
In another preferred embodiment, the sequence of the Z is as shown in SEQ ID NO: 6;

as shown in residues 440-663 of SEQ ID NO. 7, or
as shown in residues 440-663 of SEQ ID NO. 15.

In another preferred embodiment, the first polypeptide chain and the second polypeptide chain comprise an amino acid sequence as shown in any one of SEQ ID NOs. 7-15.
In another preferred embodiment, the first polypeptide chain and the second polypeptide chain comprise an amino acid sequence as shown in any one of SEQ ID NOs. 9, 10, 11, 15.
In another preferred embodiment, the fusion protein is a homodimer and comprises an amino acid selected from the group consisting of: SEQ ID NO. 9, 10, 11, 15.
In another preferred embodiment, the fusion protein can inhibit the C3 signaling pathway.
In another preferred embodiment, the fusion protein demonstrated an inhibitory effect on the complement activation pathway in a rat passive Heymann nephritis (PHN) model. The urinary protein amount in its administration group was significantly lower than that in the model group, and showed a certain dose-dependency.
In another preferred embodiment, the fusion protein demonstrated an inhibitory effect on the complement pathway in cynomolgus monkeys with spontaneous periodontitis, significantly inhibiting the disease progression of periodontitis in cynomolgus monkeys.
In a second aspect of the present invention, there is provided a nucleic acid molecule, encoding the fusion protein according to the first aspect of the present invention.
In a third aspect of the present invention, there is provided an expression vector, comprising the nucleic acid molecule according to the second aspect of the present invention.
In a preferred embodiment, the expression vector comprises: bacterial plasmids, phages, yeast plasmids, plant cell viruses, mammalian cell viruses such as adenoviruses, retroviruses, or other vectors.
In a fourth aspect of the present invention, there is provided a host cell, comprising the expression vector according to the third aspect of the present invention or capable of expressing the fusion protein according to the first aspect of the present invention.
In a fifth aspect of the present invention, there is provided a method for preparing the fusion protein according to the first aspect of the present invention, comprising the steps of: synthesizing the fusion protein according to the first aspect of the present invention, and/or, culturing the host cell according to the fourth aspect of the present invention under conditions expressing the fusion protein according to the first aspect of the present invention.
In a sixth aspect of the present invention, there is provided a pharmaceutical composition, comprising the fusion protein according to the first aspect of the present invention and optionally a pharmaceutically acceptable carrier.
In a preferred embodiment, the pharmaceutical composition is a liquid formulation.
In a preferred embodiment, the pharmaceutical composition is an injection or an injectable formulation.
In a seventh aspect of the present invention, there is provided a use of the fusion protein according to the first aspect of the present invention or the pharmaceutical composition according to the sixth aspect of the present invention in the manufacture of a medicament, the medicament being for preventing or treating a disease associated with complement activation.
In a preferred embodiment, the disease associated with complement activation is a disease of excessive complement activation.
In a preferred embodiment, the disease associated with complement activation is a C3 signaling pathway-related

disease.

In a preferred embodiment, the fusion protein or pharmaceutical composition can inhibit, alleviate, ameliorate, or reduce pathological symptoms caused by excessive complement activation, for example 1) erythrocyte lysis or hemolysis; 2) elevated urinary protein levels.

In another preferred embodiment, the disease comprises paroxysmal nocturnal hemoglobinuria (PNH), atypical hemolytic uremic syndrome (aHUS), generalized myasthenia gravis (gMG), neuromyelitis optica, age-related macular degeneration (AMD), geographic atrophy (GA), autoimmune hemolytic anemia, autoimmune thrombocytopenia, aplastic anemia, systemic lupus erythematosus, rheumatoid arthritis, ankylosing spondylitis, atherosclerosis, Parkinson's disease, Alzheimer's disease (senile dementia), asthma, allergy, psoriasis, IgA nephropathy, glomerulonephritis, lupus nephritis, multiple sclerosis, acute respiratory distress syndrome, inflammatory diseases of periodontal tissues (including gingivitis and periodontitis).

In another aspect, the present invention also provides a method for preventing or treating a disease associated with complement activation using the aforementioned fusion protein or pharmaceutical composition.

In a preferred embodiment, the disease associated with complement activation is a disease of excessive complement activation.

In a preferred embodiment, the disease comprises paroxysmal nocturnal hemoglobinuria (PNH), atypical hemolytic uremic syndrome (aHUS), generalized myasthenia gravis (gMG), neuromyelitis optica, age-related macular degeneration (AMD), geographic atrophy (GA), autoimmune hemolytic anemia, autoimmune thrombocytopenia, aplastic anemia, systemic lupus erythematosus, rheumatoid arthritis, ankylosing spondylitis, atherosclerosis, Parkinson's disease, Alzheimer's disease (senile dementia), asthma, allergy, psoriasis, IgA nephropathy, glomerulonephritis, lupus nephritis, multiple sclerosis, acute respiratory distress syndrome, inflammatory diseases of periodontal tissues (including gingivitis and periodontitis).

In an eighth aspect of the present invention, there is provided a method for preventing or treating a disease associated with complement activation, comprising administering a pharmaceutically effective amount of the fusion protein according to the first aspect of the present invention or a pharmaceutical composition comprising the fusion protein to a subject in need thereof.

It should be understood that each of the above technical features of the present invention and each of the technical features specifically described below (e.g., in the Examples) can be combined with each other within the scope of the present invention to thus form new or preferred technical solutions, which will not be repeated here one by one due to space limitation.

## BRIEF DESCRIPTION OF THE DRAWINGS

FIG. 1 shows the binding affinity of fusion proteins CRIg-FH-FCG4, CRIg-L(GS)-FH-FCG4, and CRIg-L-FH-FCG4 for human complement C3 as determined by ELISA.

FIG. 2 shows the binding affinity of fusion proteins CRIg-FH-FCG4, CRIg-FH-FCG4-M86Y, CRIg-FH-FCG4-Q64R, and CRIg-FH-FCG4-Q64R-M86Y for human complement C3 as determined by ELISA.

FIG. 3 shows the binding affinity of fusion proteins CRIg-FH-FCG4, CRIg-L(GS)-FH-FCG4, CRIg-L(GS)-FH-FCG4-Q64R, CRIg-L(GS)-FH-FCG4-M86Y, and CRIg-L(GS)-FH-FCG4-Q64R-M86Y for human complement C3 as determined by ELISA.

FIG. 4 shows the inhibitory effect of fusion proteins CRIg-FH-FCG4, CRIg-L(GS)-FH-FCG4, CRIg-L(GS)-FH-FCG4-M86Y, and CRIg-L(GS)-FH-FCG4-Q64R-M86Y on complement AP pathway-activated erythrocyte lysis.

FIG. 5 shows the inhibitory effect of fusion proteins CRIg-FH-FCG4, CRIg-L(GS)-FH-FCG4, CRIg-L(GS)-FH-FCG4-M86Y, and CRIg-L(GS)-FH-FCG4-Q64R-M86Y on complement CP pathway-activated erythrocyte lysis.

FIG. 6 shows the inhibitory effect of fusion proteins CRIg-L(GS)-FH-FCG4, CRIg-L(GS)-FH-FCG4-M86Y, and CRIg-L(GS)-FH-FCG4-Q64R-M86Y on complement AP pathway activation.

FIG. 7 shows the inhibitory effect of fusion proteins CRIg-L(GS)-FH-FCG4, CRIg-L(GS)-FH-FCG4-M86Y, and CRIg-L(GS)-FH-FCG4-Q64R-M86Y on complement CP pathway activation.

FIG. 8 shows the inhibitory effect of fusion proteins CRIg-FH-FCG4, CRIg-L(GS)-FH-FCG4, and CRIg-L(GS)-FH-FCG4-M86Y on complement LP pathway activation.

FIG. 9 shows the inhibitory effect of fusion proteins CRIg-FH-FCG4, CRIg-L(GS)-FH-FCG4, and CRIg-L(GS)-FH-FCG4-M86Y on complement AP pathway activation.

FIG. 10 shows the inhibitory effect of fusion proteins CRIg-FH-FCG4, CRIg-L(GS)-FH-FCG4, and CRIg-L(GS)-FH-FCG4-M86Y on complement CP pathway activation.

FIG. 11 shows the binding affinity of fusion proteins biotin-CRIg-L(GS)-FH-FCG4-M86Y and biotin-CRIg-L(GS)-FH-FCG4-YTE-M86Y for human complement C3 as determined by ELISA.

FIG. 12 shows the inhibitory effect of fusion proteins CRIg-L(GS)-FH-FCG4-M86Y and CRIg-L(GS)-FH-FCG4-YTE-M86Y on complement AP pathway-activated erythrocyte lysis.

FIG. 13 shows the inhibitory effect of fusion proteins CRIg-L(GS)-FH-FCG4-M86Y and CRIg-L(GS)-FH-FCG4-YTE-M86Y on complement CP pathway-activated erythrocyte lysis.

FIG. 14 shows the binding affinity of fusion proteins CRIg-L(GS)-FH-FCG4-M86Y and CRIg-L(GS)-FH-FCG4-YTE-M86Y for FcRn at pH 7.0 (A) and pH 6.0 (B) as determined by ELISA.

FIG. 15 shows the dose-dependent inhibition of 24-hour urinary protein in a rat PHN model by CRIg-L(GS)-FH-FCG4-YTE-M86Y.

## DETAILED DESCRIPTION

Through extensive and in-depth research and a large number of screenings, the inventors of the present invention have obtained a series of fusion proteins comprising CRIg and FH functional portions with high affinity and high complement inhibitory activity. As needed, the fusion protein of the present invention may further comprise functional enhancement domains such as an Fc domain. The preferred fusion protein has an affinity for human complement C3b increased by at least about 5-fold or more; more preferably, the fusion protein has an affinity increased by at least about 20-fold, 30-fold, 40-fold, 50-fold or more. The fusion protein of the present invention can effectively inhibit the activation of the complement AP pathway, complement CP pathway, and complement LP pathway. The preferred fusion protein has an inhibitory activity against the complement CP pathway increased by about 3-5 fold. The fusion protein of the present invention can effectively inhibit erythrocyte lysis activated by the complement AP pathway and complement CP pathway, thereby protecting erythrocytes. The fusion protein of the present invention also exhibits a high affinity for FcRn increased by about 6-20 fold, and is expected to have a longer half-life and superior pharmacokinetic properties. The present invention has been completed on this basis.

## Terms

Unless otherwise defined, all technical and scientific terms used herein have the same meaning as commonly understood by those of ordinary skill in the art to which the present invention belongs.

The term "about" may refer to a value or set that is within an acceptable error range for a particular value or set as determined by those of ordinary skill in the art, and the value or set partially depends on how the value or set is measured or determined.

As used herein, the terms "contain" or "comprise (comprising)" may be open-ended, semi-closed and closed-ended. In other words, the term also includes "consisting essentially of" or "consisting of".

Unless otherwise defined, all technical and scientific terms used herein have the same meaning as commonly understood by one of ordinary skill in the art to which this invention belongs. As used herein, when used in reference to a specifically enumerated numerical value, the term "about" means the value may vary from the enumerated value by no more than 1%. For example, as used herein, the expression "about 100" includes all values between 99 and 101 (e.g., 99.1, 99.2, 99.3, 99.4, etc.).

As used herein, the term "optional" or "optionally" means that the subsequently described event or circumstance may but need not occur. For example, "optionally comprising 1-3 antibody heavy chain variable regions" means that the specific sequence of antibody heavy chain variable region(s) may be present but is not required, and may be 1, 2, or 3.

As used herein, the terms "comprising," "having," or "including" encompass "containing," "consisting essentially of," "consisting substantially of," and "consisting of"; "consisting essentially of," "consisting substantially of," and "consisting of" are subordinate concepts of "comprising," "having," or "including."

## CRIg

CRIg was initially identified as a C3b/iC3b receptor expressed on the surface of macrophages. It is a complement membrane regulatory protein that can specifically recognize C3b and inhibit the activation of C3 convertase, thereby exerting an inhibitory effect early in the complement cascade. This inhibitory effect is directed against the alternative pathway of complement. The functional site of CRIg is located in its extracellular region. The CRIg domain described in this application comprises the extracellular region of CRIg. An exemplary extracellular region of CRIg comprises the amino acid sequence shown in SEQ ID NO: 2, or comprises an amino acid sequence having at least 90% identity to the amino acid sequence shown in SEQ ID NO: 2, for example, any amino acid sequence having at least 95%, at least 96%, at least 97%, at least 98%, or at least 99% identity.

**FH (Factor H)**

FH is a complement regulatory protein whose main function is to regulate the alternative pathway of the complement system. The sequence of FH contains several highly conserved motifs called short consensus repeats (SCRs). Each SCR consists of approximately 60 amino acids, and the functional properties of FH are localized to the SCRs. As used herein, the "FH domain" includes FH or variants thereof. Typically, FH comprises the SCR1-5 domains. In certain instances, FH may comprise other SCR fragments. The FH domain described in this application may include the full-length FH protein or fragments thereof (e.g., one or more SCRs), as well as various variants thereof (e.g., mutants, isoforms). An exemplary FH comprises the amino acid sequence shown in SEQ ID NO: 4, or comprises an amino acid sequence having at least 90% identity to the amino acid sequence shown in SEQ ID NO: 4, for example, any amino acid sequence having at least 95%, at least 96%, at least 97%, at least 98%, or at least 99% identity.

**Fusion Protein**

The term "fusion protein" refers to a new polypeptide sequence obtained by fusing two or more identical or different polypeptide sequences. The term "fusion" refers to direct linkage via peptide bonds or linkage via one or more linkers (peptide linkers). The term "linker (peptide linker)" refers to a short peptide that can connect two polypeptide sequences, generally having a length of 1-30 amino acids.

Preferably, the peptide linker is a flexible peptide linker. Examples of appropriate linkers include single glycine (Gly) or serine (Ser) residues, and the identifications and sequences of amino acid residues in the linker can vary depending on the type of secondary structure elements to be achieved in the linker.

As used herein, the term "bifunctional fusion protein" refers to a fusion protein capable of binding to two different target proteins with distinct specificities. The bifunctional fusion protein of the present invention binds CRIg and FH, wherein the CRIg domain variant of the bifunctional fusion protein is derived from a CRIg protein.

**Variant**

The term "variant" refers to a peptide comprising at least one amino acid substitution, deletion, or insertion relative to a wild-type or naturally occurring peptide. The fusion protein of the present invention also includes conservative variants thereof. Conservative amino acid mutation methods can be found in Table A.

Table A

| Initial residues | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr;Trp | Tyr |
| Pro (P) | Ala | Ala |
| Ser (S) | Thr | Thr |
| Thr (T) | Ser; Val | Ser |

(continued)

| Initial residues | Representative substitution | Preferred substitution |
|---|---|---|
| Trp (W) | Tyr; Phe | Tyr |
| Tyr (Y) | Trp; Phe; Thr; Ser | Phe |
| Val (V) | Ile; Leu; Met; Phe; Ala | Leu |

As used herein, the "CRIg domain variant" is derived from a CRIg domain, preferably comprising one or more amino acid mutations, substitutions, or deletions. For example, relative to the CRIg domain shown in SEQ ID NO: 2, it comprises a mutation selected from the group consisting of: Q64R, M86Y, or a combination of Q64R and M86Y.

As used herein, the "Fc domain variant" is derived from an IgG Fc domain. Said IgG Fc domain may be mutated to obtain a desired spatial structure, such as forming a knob-hole structure to better form dimers; or it may comprise the S228P mutation to improve the stability of IgG4 molecules; or mutations that enhance binding affinity to FcRn, such as the Fc domain variant comprising M252Y/S254T/T256E.

**Complement Activation-Related Diseases**

The term "complement activation-related diseases" primarily refers to diseases related to abnormal or excessive activation of complement, which may trigger inflammatory responses, as well as abnormal phagocytic effects and/or cytolytic effects. Diseases include all diseases and pathological conditions whose pathogenesis involves abnormal or excessive activation of the complement system, including diseases and pathological conditions that benefit from C3 convertase inhibition, including diseases and pathological conditions that benefit from alternative pathway complement inhibition. Complement-related diseases include, but are not limited to, inflammatory diseases and autoimmune diseases, covering acute inflammations such as ocular diseases and periodontal diseases, to chronic diseases such as cancer, autoimmune diseases, neurodegenerative diseases, kidney diseases, and chronic hemolytic diseases. Said diseases include, for example, C3-related nephropathy and TMA-related diseases. Said diseases include, for example, paroxysmal nocturnal hemoglobinuria (PNH), atypical hemolytic uremic syndrome (aHUS), generalized myasthenia gravis (gMG), neuromyelitis opticas, age-related macular degeneration (AMD), geographic atrophy (GA), autoimmune hemolytic anemia, autoimmune thrombocytopenia, aplastic anemia, systemic lupus erythematosus, rheumatoid arthritis, ankylosing spondylitis, atherosclerosis, Parkinson's disease, Alzheimer's disease (senile dementia), asthma, allergies, psoriasis, IgA nephropathy, glomerulonephritis, lupus nephritis, multiple sclerosis, acute respiratory distress syndrome, and inflammatory diseases of periodontal tissues (including gingivitis and periodontitis).

The pathogenesis of aHUS mainly involves a triggering event (such as infection or pregnancy) leading to uninhibited, sustained activation of the complement alternative pathway, resulting in membrane attack complex formation, which in turn causes renal endothelial damage, coagulation cascade activation, and renal arteriolar microthrombosis, subsequently leading to clinical manifestations such as microangiopathic hemolytic anemia, thrombocytopenia, and acute renal failure. Some aHUS patients have gene mutations in complement-related factors (such as C3). Currently marketed drugs for treating aHUS include Eculizumab and Crovalimab, which work by selectively inhibiting the activation of terminal complement component C5.

PNH is a hemolytic anemia caused by autoimmune abnormalities in the patient's body. It is a rare disease resulting from acquired gene mutations in hematopoietic stem cells, leading to the loss of the protective cell surface proteins CD55 and CD59. Normally, CD55 and CD59 effectively defend against attacks from the complement system. When CD55 and CD59 proteins are missing, red blood cells lose their self-protection ability, ultimately leading to red blood cell lysis due to excessive activation of the complement system, thereby causing the onset of PNH. Currently marketed drugs for treating PNH include the C5 inhibitors Eculizumab and Crovalimab, as well as the C3 inhibitor Pegcetacoplan (a short peptide).

Gingivitis and periodontitis are prevalent inflammatory diseases of periodontal tissues. Uncontrolled complement activation and the resulting chronic gingival inflammation are hallmarks of periodontal disease. Existing literature has reported that C3 inhibitors such as AMY-101 can help reduce inflammatory bone loss in periodontitis. C3-targeted complement therapy can be considered a valuable adjunct to non-surgical periodontal treatment (C3 Targeted Complement Therapy for Chronic Periodontitis - A Scoping Review; 10.4103/jispcd.jispcd_161_22). Results from a Phase II clinical trial (NCT0394444) of AMY-101 (a cyclic peptide) for the treatment of periodontitis and gingivitis showed that the study met its primary and key secondary endpoints. Compared to placebo, AMY-101 demonstrated statistically significant and clinically meaningful efficacy in eliminating periodontal inflammation, with good safety and tolerability (Phase IIa clinical trial of complement C3 inhibitor AMY-101 in adults with periodontal inflammation; 10.1172/JCI152973).

Preventing and/or treating complement activation-related diseases includes reducing the risk of disease occurrence, reducing the likelihood of clinical symptoms associated with the disease, reducing the severity of the disease,

and inhibiting disease progression.

**Sequence Homology**

Methods for determining sequence homology known to those of ordinary skill in the art include, but are not limited to: Computational Molecular Biology, Lesk, A.M., Ed., Oxford University Press, New York, 1988; Biocomputing: Informatics and Genome Projects, Smith, D.W., Ed., Academic Press, New York, 1993; Computer Analysis of Sequence Data, Part I, Griffin, A.M. and Griffin, H.G., Eds., Humana Press, New Jersey, 1994; Sequence Analysis in Molecular Biology, von Heinje, G., Academic Press, 1987; and Sequence Analysis Primer, Gribskov, M. and Devereux, J., Eds., M Stockton Press, New York, 1991; and Carillo, H. and Lipman, D., SIAM J. Applied Math., 48:1073 (1988). Preferred methods for determining identity are designed to give the largest match between the sequences being tested. Methods for determining identity are compiled in publicly available computer programs. Preferred computer program methods for determining identity between two sequences include, but are not limited to: the GCG program package (Devereux, J. et al., 1984), BLASTP, BLASTN, and FASTA (Altschul, S.F. et al., 1990). The BLASTX program is publicly available from NCBI and other sources (BLAST Manual, Altschul, S. et al., NCBI NLM NIH Bethesda, Md. 20894; Altschul, S. et al., 1990). The well-known Smith Waterman algorithm may also be used to determine identity.

**Nucleic Acids Encoding and Expression Vectors**

The present invention also provides nucleic acid molecules encoding the aforementioned fusion proteins. The nucleic acids of the present invention can be in DNA or RNA form. The DNA form includes cDNA, genomic DNA or synthetic DNA. The DNA may be single-stranded or double-stranded. The DNA may be a coding or non-coding strand. The sequence of the DNA molecule encoding the fusion protein of the present invention can be obtained using conventional techniques, such as PCR amplification. Furthermore, relevant sequences can be synthesized by artificial synthesis.

The present invention further relates to vectors comprising appropriate DNA sequences as described above, as well as appropriate promoters or control sequences. These vectors can be used to transform appropriate host cells to enable them to express the protein. The vector is a conventional expression vector in the art, referring to an expression vector comprising appropriate regulatory sequences, such as promoter sequences, terminator sequences, polyadenylation sequences, enhancer sequences, marker genes and/or sequences, and other appropriate sequences. The expression vector can be a virus or plasmid, such as an appropriate phage or phagemid. For more technical details, please refer to, for example, Sambrook et al., Molecular Cloning: A Laboratory Manual, Second Edition, Cold Spring Harbor Laboratory Press, 1989. Many known techniques and protocols for nucleic acid manipulation can be found in Current Protocols in Molecular Biology, Second Edition, edited by Ausubel et al. The expression vector of the present invention is preferably pcDNA3.4, pDR1, pcDNA3.1(+), pcDNA3.1/ZEO(+), pDHFR, pcDNA4, pDHFF, pGM-CSF, or pCHO 1.0.

In the present invention, the term "host cell" refers to various conventional host cells in the art, as long as they allow stable self-replication of the vector and effective expression of the carried polynucleotide molecule. The host cells include prokaryotic expression cells and eukaryotic expression cells. The host cells preferably include: COS, CHO, NS0, sf9, sf21, DH5$\alpha$, BL21(DE3), TG1, BL21(DE3), 293F, or 293E cells.

**Pharmaceutical Compositions and Applications**

The present invention also provides a pharmaceutical composition. Preferably, the composition is a pharmaceutical composition comprising the aforementioned fusion protein and a pharmaceutically acceptable carrier. Typically, these substances may be formulated in a non-toxic, inert, and pharmaceutically acceptable aqueous carrier medium, with the pH typically of about 5-8, and preferably about 6-8, although the pH may vary depending on the nature of the substance to be formulated and the condition to be treated. The formulated pharmaceutical composition can be administered via conventional routes, including (but not limited to): intravenous injection, intravenous infusion, subcutaneous injection, local injection, intramuscular injection, intratumoral injection, intraperitoneal injection (e.g., intraperitoneally), intracranial injection, or intracavitary injection. Pharmaceutically acceptable carriers include (but are not limited to): saline, buffers, glucose, water, glycerol, ethanol, and combinations thereof. The pharmaceutical formulation should match the mode of administration. The pharmaceutical composition of the present invention may be prepared into an injectable form, for example, by conventional methods using normal saline or aqueous solutions containing glucose and other excipients.

The present invention is further described below in conjunction with specific examples. It should be understood that these examples are intended only to illustrate the present invention, rather than limiting the scope of the present invention. In the following examples, experimental methods without specified detailed conditions are generally carried out under conventional conditions as described in Sambrook et al., Molecular Cloning: A Laboratory Manual (New York: Cold Spring Harbor Laboratory Press, 1989), or as recommended by the manufacturers. Unless otherwise stated, percentages and parts are calculated by weight.

**The main advantages of the present invention include:**

(1) The fusion protein of the present invention has higher affinity for the key human complement pathway protein C3b; effectively inhibits the activation of multiple complement pathways; effectively inhibits complement pathway activation-induced hemolysis; and also exhibits high affinity for FcRn.

(2) The fusion protein of the present invention exhibits approximately 6-20 times higher affinity for FcRn, and is expected to have a longer half-life and superior pharmacokinetic properties.

(3) The CRIg-FH fusion protein of the present invention is simultaneously engineered for the treatment of diseases related to abnormal activation of the complement system; CRIg - FH primarily targets the key protein C3b in the mid and terminal stages of complement pathway activation, and C3b is transiently produced in the body with a short half -life. The CRIg-FH fusion protein obtained in the present invention may have lower side effects and superior therapeutic effects compared to currently marketed inhibitors targeting C3 and/or C5 proteins.

The present invention is further described below in conjunction with specific examples. It should be understood that these examples are intended only to illustrate the present invention, rather than limiting the scope of the present invention. In the following examples, experimental methods without specified detailed conditions are generally carried out under conventional conditions as described in Sambrook et al., Molecular Cloning: A Laboratory Manual (New York: Cold Spring Harbor Laboratory Press, 1989), or as recommended by the manufacturers. Unless otherwise stated, percentages and parts are calculated by weight.

**Example 1 Activity Assay of Humanized Fusion Protein**

**1.1 Preparation of Humanized Fusion Protein**

CRIg-L-FH-IgG4 is a fusion protein consisting of the CRIg extracellular domain (G19-K137, NCBI Gene ID: NM_007268.3), a Linker, Factor H short consensus repeat domains (SCRs1-5, E19-K323, NCBI Gene ID: NM_000186.4), and the human IgG4 constant region Fc. The CRIg-L-FH-IgG4 pcDNA3.4 plasmid was obtained by gene synthesis and cloning. The CRIg extracellular domain gene has a full length of 357 bp, encoding 119 amino acids, with the nucleotide sequence shown in SEQ ID NO: 1 and the amino acid sequence shown in SEQ ID NO: 2; the Linker sequence is $L(Ser_1Gly_4)_3$: $(SG_4)_3$; the Factor H short consensus repeat domain gene has a full length of 915 bp, encoding 305 amino acids, with the nucleotide sequence shown in SEQ ID NO: 3 and the amino acid sequence shown in SEQ ID NO: 4; the human IgG4 constant region Fc gene has a full length of 675 bp, encoding 224 amino acids, with the nucleotide sequence shown in SEQ ID NO: 5 and the amino acid sequence shown in SEQ ID NO: 6.

Using the CRIg-L-FH-IgG4 plasmid as a template, primers were designed to mutate the serine at position 228 in the human IgG4 constant region Fc sequence to proline (S228P) by PCR, resulting in a construct named CRIg-L-FH-FCG4 (with the mutated Fc region designated as FCG4). The CRIg extracellular domain and Factor H short consensus repeat domain were individually connected to FCG4 by molecular cloning techniques to construct the CRIg-FCG4-pcDNA3.4, FH-FCG4-pcDNA3.4, and FH-L(GS)-CRIg-FCG4-pcDNA3.4 plasmids.

The amino acid sequence of CRIg-L-FH-FCG4 is shown in SEQ ID NO: 7.

Using the CRIg-L-FH-FCG4 plasmid as a template, primers were designed, and the Linker in the CRIg-L-FH-FCG4 sequence was deleted by PCR, yielding the CRIg-FH-FCG4 plasmid.

Using the CRIg-L-FH-FCG4 plasmid as a template, primers were designed, and the Linker sequence $L(Ser_1Gly_4)_3$ was replaced by $L(Gly_4Ser_1)_3$: $(G_4S)_3$ via PCR to obtain the CRIg-L(GS)-FH-FCG4 plasmid, with the amino acid sequence shown in SEQ ID NO: 8.

Using the CRIg-L(GS)-FH-FCG4 plasmid as a template, primers were designed, and the glutamine at position 64 of the CRIg sequence was mutated to arginine (Q64R) by PCR, yielding the CRIg-L(GS)-FH-FCG4-Q64R plasmid. The amino acid sequence of CRIg-L(GS)-FH-FCG4-Q64R is shown in SEQ ID NO: 9.

Using the CRIg-L(GS)-FH-FCG4 plasmid as a template, primers were designed, and the methionine at position 86 of the CRIg sequence was mutated to tyrosine (M86Y) by PCR, yielding CRIg-L(GS)-FH-FCG4-M86Y. The amino acid sequence of CRIg-L(GS)-FH-FCG4-M86Y is shown in SEQ ID NO: 10.

Using the CRIg-L(GS)-FH-FCG4 plasmid as a template, primers were designed to simultaneously mutate glutamine at position 64 to arginine (Q64R) and methionine at position 86 to tyrosine (M86Y) in the CRIg sequence by PCR, yielding CRIg-L(GS)-FH-FCG4-Q64R-M86Y. The amino acid sequence of CRIg-L(GS)-FH-FCG4-Q64R-M86Y is shown in SEQ ID NO: 11.

Using the CRIg-FH-FCG4 plasmid as a template, primers were designed, and the glutamine at position 64 and the methionine at position 86 of the CRIg sequence were mutated to arginine (Q64R) and tyrosine (M86Y), respectively,

and both amino acid sites were mutated by PCR, yielding three plasmids: CRIg-FH-FCG4-Q64R, CRIg-FH-FCG4-M86Y, and CRIg-FH-FCG4-Q64R-M86Y, with amino acid sequences shown in SEQ ID NO: 12, 13, and 14, respectively.

Each pcDNA3.4 expression vector encoding a fusion protein was transfected into HEK-293F cells, and the cells were cultured for 6 days to allow protein expression, and the cell supernatants were collected. Each fusion protein was obtained by Protein A purification.

The molecular weight of each expressed protein was confirmed to be as expected by SDS - PAGE electrophoresis and SEC-HPLC, with antibody purity >95%. The proteins were quantified, aliquoted, and stored at -80°C for future use.

**1.2 Determination of Binding Affinity of Each Fusion Protein for Human Complement C3 by ELISA**

C3-his protein (purchased from Sino Biological Inc., Cat# 13182-H08H) was diluted to 2 μg/mL and coated onto an ELISA plate, 100 μL/well, and incubated overnight at 4°C. The plate was washed three times with PBST (PBS containing 0.05% Tween20), blocked with 2% BSA prepared in PBST, 200 μL/well, for 2 hours at room temperature. After washing twice with PBST, 50 mM $NiCl_2$ was diluted to a concentration of 5 mM using 1% BSA prepared in PBST. The test proteins were serially diluted 5-fold in 1% BSA containing 5 mM $NiCl_2$, with the highest concentration being 200 nM, across 8 dilution gradients. The dilutions were added to the ELISA wells, 100 μL/well, and incubated for 1 hour at room temperature, with each sample tested in duplicate. The plate was washed three times with PBST. The secondary antibody (Anti-Human IgG (Fc specific)-Peroxidase antibody, purchased from Sigma-Aldrich LLC, Cat#: A0170) was diluted at an appropriate ratio in 1% BSA prepared in PBST, added to the ELISA wells, 100 μL/well, and incubated for 1 hour at room temperature. After washing three times with PBST, TMB substrate solution was added, 100 μL/well, and color development proceeded until the expected color was reached. The color development reaction was stopped with 2M $H_2SO_4$, 50 μL/well. The reaction solutions were mixed thoroughly by shaking, and the OD 450 nm was measured using a microplate reader. Data were analyzed to calculate the $EC_{50}$.

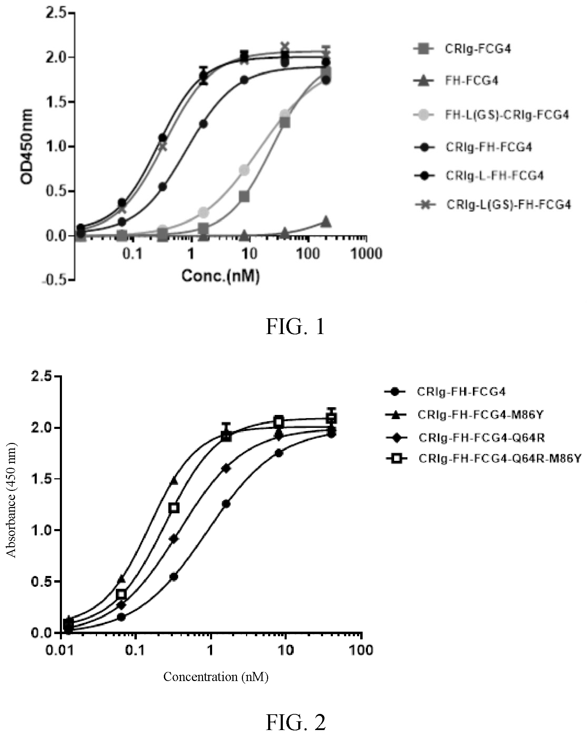

The experimental results are shown in Figure 1 and Table 1. The binding affinity of CRIg - L(GS)-FH-FCG4 for human complement C3 was significantly higher than that of the single fusion proteins CRIg-FCG4 and FH-FCG4, and also significantly better than that of the bifunctional fusion proteins CRIg-FH-FCG4 and FH-L(GS)-CRIg-FCG4. The binding affinity of CRIg-L(GS)-FH-FCG4 for human complement C3 was similar to that of CRIg-L-FH-FCG4.

Table 1: $EC_{50}$ values for the affinity of fusion proteins for C3-His protein

| Sample | Mean (nM) | SD |
|---|---|---|
| CRIg-FCG4 | 24.455 | 1.690 |
| FH-FCG4 | \ | \ |
| FH-L(GS)-CRIg-FCG4 | 14.500 | 1.754 |
| CRIg-FH-FCG4 | 0.903 | 0.037 |
| CRIg-L-FH-FCG4 | 0.275 | 0.001 |
| CRIg-L(GS)-FH-FCG4 | 0.345 | 0.028 |

As shown in Figure 2 and Table 2, mutations Q64R and M86Y in CRIg significantly improved the binding affinity of the fusion proteins for complement C3. The $EC_{50}$ value for C3-His protein binding of CRIg-FH-FCG4-Q64R was decreased by approximately 2.5-fold compared to that of CRIg-FH-FCG4. The $EC_{50}$ value for C3-His protein binding of CRIg-FH-FCG4-Q64R-M86Y was increased by approximately 3.5-fold compared to that of CRIg-FH-FCG4. The $EC_{50}$ value for C3-His protein binding of CRIg-FH-FCG4-M86Y was increased by approximately 5.1-fold compared to that of CRIg-FH-FCG4.

Table 2: $EC_{50}$ values for the affinity of fusion protein CRIg-FH-FCG4 and its mutant proteins for C3-His

| Sample | $EC_{50}$ (nM) | SD |
|---|---|---|
| CRIg-FH-FCG4 | 0.903 | 0.037 |
| CRIg-FH-FCG4-M86Y | 0.176 | 0.029 |
| CRIg-FH-FCG4-Q64R | 0.366 | 0.038 |
| CRIg-FH-FCG4-Q64R-M86Y | 0.259 | 0.031 |

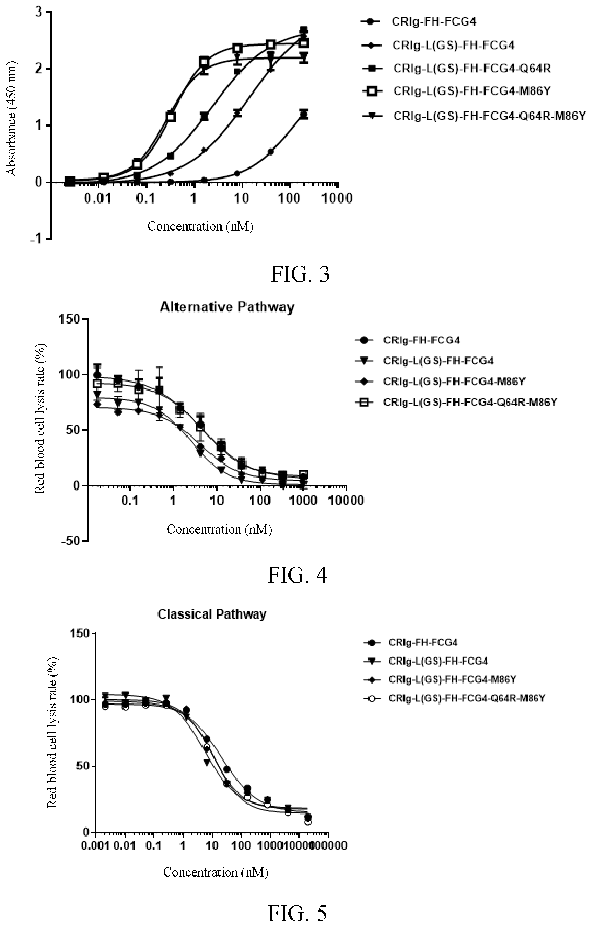

As shown in Figure 3 and Table 3, the introduction of Q64R and M86Y mutations into CRIg-L(GS)-FH-FCG4 also significantly improved its binding affinity for complement C3. When the coating concentration of C3-his protein was reduced to 1 μg/mL, the $EC_{50}$ value for C3-His protein binding of CRIg-L(GS)-FH-FCG4-Q64R was decreased by approximately 5.8-fold compared to that of CRIg-L(GS)-FH-FCG4. The $EC_{50}$ value for C3-His protein binding of CRIg-L(GS)-FH-FCG4-Q64R-M86Y was increased by approximately 52.8-fold compared to that of CRIg-L(GS)-FH-FCG4. The $EC_{50}$ value for C3-His protein binding of CRIg-L(GS)-FH-FCG4-M86Y was increased by approximately 39.8-fold compared to that of CRIg-L(GS)-FH-FCG4.

Table 3: $EC_{50}$ values for the affinity of fusion protein CRIg-L(GS)-FH-FCG4 and its mutant proteins for C3-His

| Sample | Mean (nM) | SD |
|---|---|---|
| CRIg-FH-FCG4 | 109.000 | 4.101 |
| CRIg-L(GS)-FH-FCG4 | 14.245 | 4.137 |
| CRIg-L(GS)-FH-FCG4-Q64R | 2.462 | 0.507 |
| CRIg-L(GS)-FH-FCG4-M86Y | 0.358 | 0.030 |
| CRIg-L(GS)-FH-FCG4-Q64R-M86Y | 0.270 | 0.018 |

**Example 2 Fusion proteins protect human red blood cells from complement-mediated hemolysis induced via the alternative pathway and the classical pathway**

**2.1 Fusion proteins protect human red blood cells from hemolysis induced by activation of the complement alternative pathway (AP)**

Human red blood cells (purchased from Shanghai Aoneng Biotechnology Co., Ltd., Cat# FTS-RBC-3) were mixed with Alsever's solution (Sigma, A3551) at a 1:1 volume ratio, mixed well, and stored at 4°C. For use, the cells were counted; $3 \times 10^9$ cells were taken and added to 10 mL of 0.9% saline, mixed well, centrifuged at 2000 rpm for 10 min, and the supernatant was removed. The above steps were repeated, and the cells were washed three times. The cells were resuspended in AP buffer (DPB+5.5mM $MgCl_2$+0.15mM $CaCl_2$+8mM EGTA), and the total cell count was determined using trypan blue. The cell density was adjusted to 4E8/mL, and 50 μL was added per well to a 96-well cell culture plate. Normal human serum (Normal Human Serum, purchased from Shanghai Aoneng Biotechnology Co., Ltd., Cat# FTS-S-M-A-50) was used to serially dilute each fusion protein 5-fold, with the highest concentration being 10000 nM (final concentration 5000 nM), across 11 dilution gradients. The dilutions were added to the red blood cells, 100 μL/well. The NC wells received 100 μL of inactivated human serum, and the PC wells received 100 μL of $ddH_2O$. Each sample was tested in duplicate. The plate was incubated at 37°C for 15 min. CVF (C3- and C5-activating cobra venom factor, purchased from Quidel Corporation, Cat# A600) was diluted to 40 μg/mL (final concentration 10 μg/mL) using AP buffer, and 50 μL was added per well. After mixing, the plate was incubated at 37°C for 60 min. The plate was centrifuged at 3000 rpm for 3 min, 100 μL of supernatant was transferred to a new 96-well cell culture plate, and the absorbance was measured at 415 nm using a multifunctional microplate reader. Data were analyzed.

$$\text{Red blood cell lysis rate (\%)} = (\text{Sample} - \text{NC}) / (\text{Sample (0 nM)} - \text{NC}) * 100.$$

The experimental results are shown in Figure 4 and Table 4. The inhibitory effect of CRIg - L(GS)-FH-FCG4-M86Y on AP pathway-activated red blood cell lysis was comparable to that of CRIg-L(GS)-FH-FCG4 and superior to that of CRIg-FH-FCG4 and CRIg-L(GS)-FH-FCG4-Q64R-M86Y.

Table 4: $IC_{50}$ values for the inhibition of complement alternative pathway-activated red blood cell lysis by each fusion protein

| Sample | Mean (nM) | SD |
|---|---|---|
| CRIg-FH-FCG4 | 4.213 | 0.438 |
| CRIg-L(GS)-FH-FCG4 | 2.459 | 0.018 |
| CRIg-L(GS)-FH-FCG4-M86Y | 3.911 | 0.227 |
| CRIg-L(GS)-FH-FCG4-Q64R-M86Y | 4.561 | 0.427 |

**2.2 Fusion proteins protect human red blood cells from hemolysis induced by activation of the complement classical pathway (CP)**

Human red blood cells (purchased from Shanghai Aoneng Biotechnology Co., Ltd., Cat# FTS-RBC-3) were mixed with Alsever's solution (Sigma, A3551) at a 1:1 volume ratio, mixed well, and stored at 4°C. For use, the cells were counted; $3 \times 10^9$ cells were taken and added to 10 mL of 0.9% saline, centrifuged at 2000 rpm for 10 min, and the supernatant was removed. The above steps were repeated, and the cells were washed three times. The cells were resuspended in CP buffer (DPBS+0.5mM MgCl2+0.15mM CaCl2), and the total cell count was determined using trypan blue. The cell density was adjusted to 4E8/mL, and 50 μL was added per well to a 96-well cell culture plate. Normal human serum (Normal Human Serum, purchased from Shanghai Aoneng Biotechnology Co., Ltd., Cat# FTS-S-MA-50) was diluted to a 20% concentration (final concentration 10%) using CP buffer. The fusion proteins were serially diluted 5-fold in CP buffer containing 20% human serum, with the highest concentration being 40000 nM (final concentration 20000 nM), across 11 dilution gradients. The dilutions were added to the red blood cells, 100 μL/well. The NC wells received 100 μL of 20% inactivated human serum, and the PC wells received 100 μL of $ddH_2O$. Each sample was tested in duplicate. After mixing, the plate was incubated at 37°C for 15 min. Human Red Blood Cell Antibody (anti-human erythrocyte antibody, purchased from Rockland Immunochemicals, Cat# 209-4139) was diluted to 600 μg/mL (final concentration 150 μg/mL) using CP buffer, and 50 μL was added per well. After mixing, the plate was incubated at 37°C for 30 min. The plate was centrifuged at 3000 rpm for 3 min, 100 μL of supernatant was transferred to a new 96-well cell culture plate, and the absorbance was measured at 415 nm using a multifunctional microplate reader. Data were analyzed.

$$\text{Red blood cell lysis rate (\%)} = (\text{Sample} - \text{NC}) / (\text{Sample (0 nM)} - \text{NC}) * 100.$$

The experimental results are shown in Figure 5 and Table 5. Based on the dose-response curves for inhibition of classical complement pathway-activated red blood cell lysis, the inhibitory effects of CRIg-L(GS)-FH-FCG4-M86Y, CRIg-L(GS)-FH-FCG4-Q64R-M86Y, and CRIg-L(GS)-FH-FCG4 on classical pathway-activated red blood cell lysis were similar and superior to that of CRIg-FH-FCG4.

Table 5: $IC_{50}$ values for the inhibition of complement classical pathway-activated red blood cell lysis by each fusion protein

| Sample | Mean (nM) | SD |
|---|---|---|
| CRIg-FH-FCG4 | 19.965 | 0.361 |
| CRIg-L(GS)-FH-FCG4 | 5.445 | 0.021 |
| CRIg-L(GS)-FH-FCG4-M86Y | 9.937 | 1.800 |
| CRIg-L(GS)-FH-FCG4-Q64R-M86Y | 12.135 | 1.520 |

**Example 3 Inhibition of complement alternative pathway and classical pathway activation by fusion proteins**

The inhibitory effects of the fusion proteins on complement classical pathway and alternative pathway activation were detected using commercially available kits WIESLAB® Complement System Classical Pathway (COMPL CP310) and WIESLAB® Complement Alternative Pathway (COMPL AP330) purchased from Svar, respectively.

**3.1 Determination of the inhibitory effect of fusion proteins on the complement alternative pathway (AP) by ELISA**

ELISA detection was performed using the commercially available kit WIESLAB® Complement Alternative Pathway (COMPL AP330). The kit was equilibrated to room temperature before use. The 30× wash buffer was diluted with $ddH_2O$. PC was dissolved on ice with 200 μL $ddH_2O$, aliquoted, and stored at -80°C, to avoid repeated freeze-thaw cycles. PC and NC were diluted 19-fold with Diluent AP (15 μL + 270 μL). Normal human serum (Normal Human Serum, purchased from Shanghai Aoneng Biotechnology Co., Ltd., Cat# FTS-S-M-A-50) was diluted to 5% using Diluent AP. The test proteins were serially diluted 3-fold in Diluent AP containing 5% human serum, with the highest concentration being 10 nM, across 8 dilution gradients. The dilutions were added to the LPS-coated ELISA plate at 100 μL/well and incubated at 37°C for 70 min. Each sample was tested in duplicate. The plate was washed three times with 300 μL/well of wash buffer and blotted dry on absorbent paper to remove excess liquid. Conjugate containing alkaline phosphatase-labelled antibodies to C5b-9 was added at 100 μL/well and incubated at room temperature for 30 min. The plate was washed three times with 300 μL/well of wash buffer and blotted dry on absorbent paper to remove excess liquid. Substrate solution

was added at 100 μL/well and incubated at room temperature for 30 min. The reaction was stopped by adding 5 mM EDTA at 100 μL/well, and the absorbance was measured at 405 nm using a multifunctional microplate reader. Data were analyzed.

$$\text{Data processing method: Inhibition rate (\%BL)} = (\text{Sample} - \text{BK}) / (\text{Sample}_{(0\ \text{nM})} - \text{BK}) * 100$$

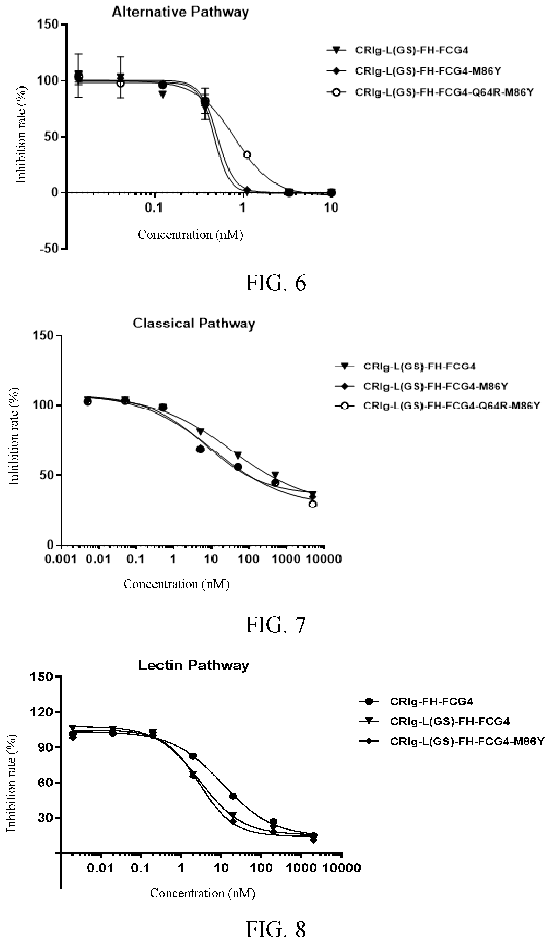

The experimental results are shown in Figure 6 and Table 6. The inhibitory activity of CRIg-L(GS)-FH-FCG4-M86Y on AP pathway activation was comparable to that of CRIg-L(GS)-FH-FCG4 and superior to that of CRIg-L(GS)-FH-FCG4-Q64R-M86Y.

Table 6: $IC_{50}$ values for the inhibition of alternative pathway activation by each fusion protein

| Sample | Mean (nM) | SD |
|---|---|---|
| CRIg-L(GS)-FH-FCG4 | 0.471 | 0.023 |
| CRIg-L(GS)-FH-FCG4-M86Y | 0.511 | 0.023 |
| CRIg-L(GS)-FH-FCG4-Q64R-M86Y | 0.808 | 0.094 |

**3.2 Determination of the inhibitory effect of fusion proteins on the complement classical pathway (CP) by ELISA**

ELISA detection was performed using the commercially available kit WIESLAB® Complement System Classical Pathway (COMPL CP310). The kit was equilibrated to room temperature before use. The 30× wash buffer was diluted with $ddH_2O$. PC was dissolved on ice with 200 μL $ddH_2O$, aliquoted, and stored at -80°C, to avoid repeated freeze-thaw cycles. PC and NC were diluted 102-fold with Diluent CP (3 μL + 303 μL). Normal human serum (Normal Human Serum, purchased from Shanghai Aoneng Biotechnology Co., Ltd., Cat# FTS-S-M-A-50) was diluted to 1% using Diluent CP. The test proteins were serially diluted 10-fold in Diluent CP containing 1% human serum, with the highest concentration being 2000 nM, across 8 dilution gradients. The dilutions were added to the IgM-coated ELISA plate at 100 μL/well and incubated at 37°C for 70 min. Each sample was tested in duplicate. The plate was washed three times with 300 μL/well of wash buffer and blotted dry on absorbent paper to remove excess liquid. Conjugate containing alkaline phosphatase-labelled antibodies to C5b-9 was added at 100 μL/well and incubated at room temperature for 30 min. The plate was washed three times with wash buffer and blotted dry on absorbent paper to remove excess liquid. Substrate solution was added at 100 μL/well and incubated at room temperature for 30 min. The reaction was stopped by adding 5 mM EDTA at 100 μL/well, and the absorbance was measured at 405 nm using a multifunctional microplate reader. Data were analyzed.

Data processing method: Inhibition rate (%BL) = (Sample - BK) / ($\text{Sample}_{(0\ \text{nM})}$ - BK) * 100

The experimental results are shown in Figure 7 and Table 7. Based on the dose-response curves, the inhibitory activities of CRIg-L(GS)-FH-FCG4-M86Y and CRIg-L(GS)-FH-FCG4-Q64R-M86Y on complement classical pathway activation were comparable and superior to that of CRIg-L(GS)-FH-FCG4.

Table 7: $IC_{50}$ values for the inhibition of classical pathway activation by each fusion protein

| Sample | Mean (nM) | SD |
|---|---|---|
| CRIg-L(GS)-FH-FCG4 | 35.115 | 0.912 |
| CRIg-L(GS)-FH-FCG4-M86Y | 7.048 | 0.272 |
| CRIg-L(GS)-FH-FCG4-Q64R-M86Y | 11.630 | 1.329 |

**Example 4 Inhibition of complement lectin pathway, alternative pathway, and classical pathway activation by fusion proteins**

Based on the results of hemolysis assays and complement activity inhibition experiments, CRIg-FH-FCG4-M86Y, which exhibited stronger binding affinity to complement C3 after mutation, was selected for further validation.

**4.1 Determination of the inhibitory effect of fusion proteins on complement lectin pathway (LP) activation by ELISA**

ELISA detection was performed using the commercially available kit WIESLAB® Complement system MBL pathway (COMPL MP320). The kit was equilibrated to room temperature before use. The 30× wash buffer was diluted with $ddH_2O$. PC was dissolved on ice with 200 μL $ddH_2O$, aliquoted, and stored at -80°C, to avoid repeated freeze-thaw cycles. PC and NC were diluted 102-fold with Diluent MP (3 μL + 303 μL). Normal human serum (Nomal Human Serum, purchased from Shanghai Aoneng Biotechnology Co., Ltd., Cat# FTS-S-M-A-50) was diluted to 1% using Diluent MP. The test proteins were serially diluted 10-fold in Diluent MP containing 1% human serum, with the highest concentration being 2000 nM, across 8 dilution gradients. The dilutions were added to the mannan-coated ELISA plate at 100 μL/well and incubated at 37°C for 70 min. Each sample was tested in duplicate. The plate was washed three times with 300 μL/well of wash buffer and blotted dry on absorbent paper to remove excess liquid. Conjugate containing alkaline phosphatase-labelled antibodies to C5b-9 was added at 100 μL/well and incubated at room temperature for 30 min. The plate was washed three times with wash buffer and blotted dry on absorbent paper to remove excess liquid. Substrate solution was added at 100 μL/well and incubated at room temperature for 30 min. The reaction was stopped by adding 5 mM EDTA at 100 μL/well, and the absorbance was measured at 405 nm using a multifunctional microplate reader. Data were analyzed.

$$\text{Data processing method: Inhibition rate (\%BL)} = (\text{Sample} - \text{BK}) / (\text{Sample}_{(0\ \text{nM})} - \text{BK}) * 100$$

The experimental results are shown in Figure 8 and Table 8. The inhibitory effect of CRIg - L(GS)-FH-FCG4-M86Y on complement lectin pathway activation was slightly superior to that of CRIg-L(GS)-FH-FCG4 and significantly superior (approximately 4-fold) to that of CRIg-FH-FCG4.

Table 8: $IC_{50}$ values for the inhibition of lectin pathway activation by each fusion protein

| Sample | Mean (nM) | SD |
|---|---|---|
| CRIg-FH-FCG4 | 10.915 | 0.856 |
| CRIg-L(GS)-FH-FCG4 | 2.881 | 0.092 |
| CRIg-L(GS)-FH-FCG4-M86Y | 2.769 | 0.064 |

**4.2 Determination of the inhibitory effect of fusion proteins on complement alternative pathway (AP) and classical pathway (CP) by ELISA**

The experimental methods referred to Examples 3.1 and 3.2.

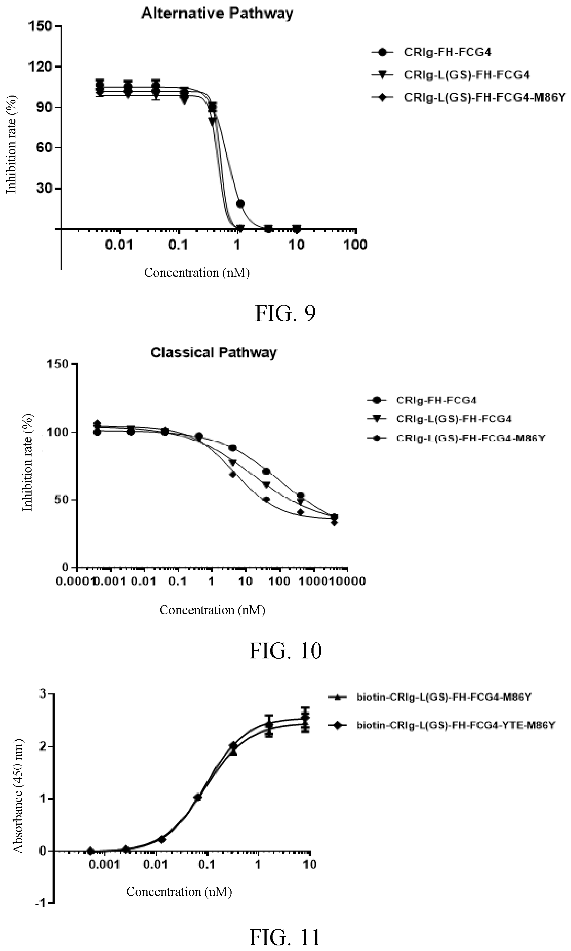

The experimental results are shown in Figure 9 and Table 9. The inhibitory activity of CRIg-L(GS)-FH-FCG4-M86Y on complement alternative pathway activation was equivalent to that of CRIg-L(GS)-FH-FCG4 and superior to that of CRIg-FH-FCG4.

Table 9: $IC_{50}$ values for the inhibition of complement alternative pathway activation by each fusion protein

| Sample | Mean (nM) | SD |
|---|---|---|
| CRIg-FH-FCG4 | 0.672 | 0.004 |
| CRIg-L(GS)-FH-FCG4 | 0.463 | 0.015 |
| CRIg-L(GS)-FH-FCG4-M86Y | 0.508 | 0.029 |

The experimental results are shown in Figure 10 and Table 10. The inhibitory activity of CRIg-L(GS)-FH-FCG4-M86Y on complement classical pathway activation was significantly superior to that of CRIg-L(GS)-FH-FCG4 and CRIg-FH-FCG4.

Table 10: $IC_{50}$ values for the inhibition of complement classical pathway activation by each fusion protein

| Sample | Mean (nM) | SD |
|---|---|---|
| CRIg-FH-FCG4 | 100.675 | 11.915 |
| CRIg-L(GS)-FH-FCG4 | 16.045 | 0.544 |

(continued)

| Sample | Mean (nM) | SD |
|---|---|---|
| CRIg-L(GS)-FH-FCG4-M86Y | 4.687 | 0.025 |

**Example 5 Preparation of the fusion protein CRIg-L(GS)-FH-FCG4-YTE-M86Y**

Studies have shown that the M252Y/S254T/T256E amino acid mutations in the Fc region of IgG antibodies can reduce the elimination rate of antibodies in circulation, enhance the binding affinity of IgG antibodies to FcRn at pH 6.0, and thereby prolong their half-life in serum.

Using the CRIg-L(GS)-FH-FCG4-M86Y pcDNA3.4 plasmid as a template, the M252Y/S254T/T256E amino acid mutations on the Fc sequence were completed by PCR, and the resulting construct was named CRIg-L(GS)-FH-FCG4-YTE -M86Y, with the amino acid sequence shown in SEQ ID NO: 15.

The fusion protein CRIg-L(GS)-FH-FCG4-YTE-M86Y pcDNA3.4 expression vector was transfected into HEK-293F cells and expressed for 6 days. The supernatant was collected, and the humanized proteins were obtained by Protein A purification. The molecular weight of the expressed fusion proteins was determined to be approximately 148 kD with purity >95% by SDS-PAGE electrophoresis and SEC-HPLC. The proteins were quantified, aliquoted, and stored at -80°C for future use.

**Example 6 Determination of the binding affinity of CRIg-L(GS)-FH-FCG4-YTE-M86Y to human complement C3 by ELISA**

Protein biotinylation: The amount of biotinylation reagent required for CRIg-L(GS)-FH-FCG4-M86Y and CRIg-L(GS)-FH-FCG4-YTE-M86Y was calculated according to the instructions of EZ-Link™ NHS-LC-LC-Biotin (purchased from Thermo Scientific™, Cat#: 21343). The proteins were mixed with the biotin reagent and incubated at room temperature for 1 hour. Excess biotinylation reagent was removed by protein ultrafiltration using PBS, and the proteins were quantified.

The C3-his protein (Complement component 3 Protein, Human, Recombinant (His Tag), purchased from Sino Biological Inc., Cat# 13182-H08H) was diluted to 2 μg/mL and coated onto an ELISA plate at 100 μL/well, and incubated overnight at 4°C. The ELISA plate was washed three times with PBST (PBS containing 0.05% Tween20) and blocked with 2% BSA in PBST at 200 μL/well for 2 hours at room temperature. After washing twice with PBST, 50 mM $NiCl_2$ was diluted to a concentration of 5 mM using 1% BSA in PBST. The proteins were serially diluted 5-fold in 1% BSA containing 5 mM $NiCl_2$, with the highest concentration being 200 nM, across 8 dilution gradients. The dilutions were added to the ELISA wells at 100 μL/well and incubated at room temperature for 1 hour. Each sample was tested in duplicate. The plate was washed three times with PBST. The secondary antibody (HRP-Streptavidin, purchased from BD Biosciences, Cat#: 554066) was diluted at a 1:8000 ratio in 1% BSA in PBST and added to the ELISA wells at 100 μL/well, followed by incubation at room temperature for 1 hour. After washing three times with PBST, TMB chromogenic solution was added at 100 μL/well and developed until the desired color was achieved. The chromogenic reaction was stopped by adding 2 M $H_2SO_4$ at 50 μL/well. The reaction mixtures were mixed thoroughly by shaking, and the OD450nm was measured using a microplate reader. Data were analyzed, and the $EC_{50}$ was calculated.

The experimental results are shown in Figure 11 and Table 11. The addition of the YTE mutation to the Fc region of CRIg-L(GS)-FH-FCG4-M86 did not alter its affinity for C3.

Table 11: $EC_{50}$ values for the binding of fusion proteins to the C3-His protein

| Sample | Mean (nM) | SD | Bottom |
|---|---|---|---|
| biotin-CRIg-L(GS)-FH-FCG4-M86Y | 0.091 | 0.007 | -0.020 |
| biotin-CRIg-L(GS)-FH-FCG4-YTE-M86Y | 0.093 | 0.014 | -0.013 |

**Example 7 Protection of human red blood cells from hemolysis induced by complement alternative pathway and classical pathway activation by CRIg-L(GS)-FH-FCG4-YTE-M86Y**

The inhibitory effect of CRIg-L(GS)-FH-FCG4-YTE-M86Y on red blood cell lysis was detected by hemolysis assay, referring to the experimental method described in Example 2.

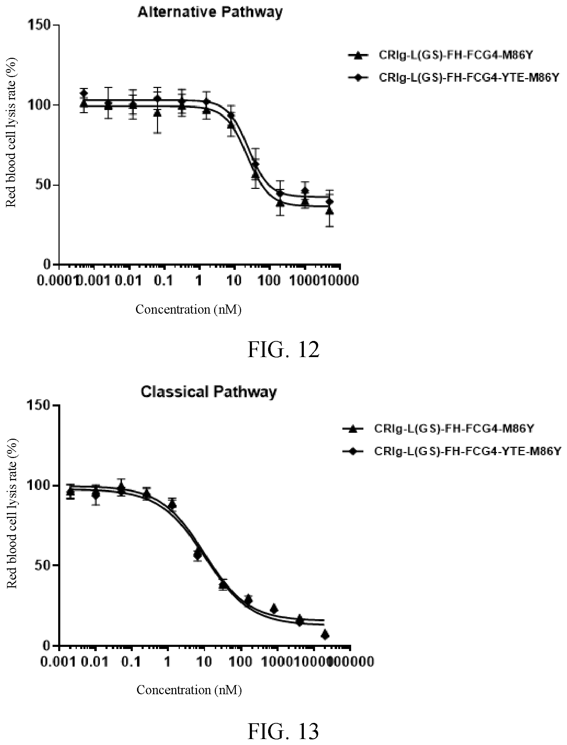

The experimental results are shown in Figure 12 and Table 12. The addition of the YTE mutation to the Fc region of CRIg-L(GS)-FH-FCG4-M86 did not alter its inhibitory effect on hemolysis induced by complement alternative pathway activation.

Table 12: $IC_{50}$ values for the inhibition of red blood cell lysis induced by complement alternative pathway activation by the fusion proteins

| Sample | Mean (nM) | SD |
|---|---|---|
| CRIg-L(GS)-FH-FCG4-M86Y | 23.545 | 1.846 |
| CRIg-L(GS)-FH-FCG4-YTE-M86Y | 25.365 | 2.425 |

Table 13: $IC_{50}$ values for the inhibition of red blood cell lysis induced by complement classical pathway activation by the fusion proteins

| Sample | Mean (nM) | SD |
|---|---|---|
| CRIg-L(GS)-FH-FCG4-M86Y | 10.293 | 2.980 |
| CRIg-L(GS)-FH-FCG4-YTE-M86Y | 10.455 | 1.464 |

**Example 8 Binding affinity of the fusion protein CRIg-L(GS)-FH-FCG4-YTE-M86Y to FcRn**

**8.1 Binding assay under pH 7.0 conditions**

The FcRn-his protein (purchased from Sino Biological Inc., Cat# CT009-H08H) was biotinylated according to the method described in Example 6.

The proteins CRIg-L(GS)-FH-FCG4-M86Y and CRIg-L(GS)-FH-FCG4-YTE-M86Y were diluted to 5 μg/mL using ELISA coating buffer and coated onto an ELISA plate at 100 μL/well. The plate was placed in a humidified chamber and incubated at 4°C for 16 hours. The ELISA plate was washed three times with pH 7.0 PBST to remove unbound antigen, blotted dry on absorbent paper to remove excess liquid, and then blocked with 2% BSA in pH 7.0 PBS at 200 μL/well for 2 hours at room temperature. The plate was washed once with pH 7.0 PBST to remove excess blocking solution, blotted dry to remove excess liquid. The biotin-FcRn-his protein was serially diluted 3-fold in 1% BSA in pH 7.0 PBST, with the highest concentration being 10000 nM, across 12 dilution gradients. The dilutions were added to the ELISA wells at 100 μL/well and incubated at room temperature for 1 hour. Each sample was tested in duplicate. Unbound or non-specifically bound primary antibody was washed away with pH 7.0 PBST. The HRP-labeled Anti-Human SA secondary antibody was diluted 1:8000 in 1% BSA in pH 7.0 PBST, added to the ELISA plate at 100 μL/well, and incubated at room temperature for 0.5 hours. The plate was washed five times with pH 7.0 PBST, blotted dry on absorbent paper to remove excess liquid. TMB chromogenic solution was added at 100 μL/well and developed to an appropriate intensity. The chromogenic reaction was stopped by adding 2M $H_2SO_4$ at 50 μL/well. The absorbance was measured at 450 nm using a multifunctional microplate reader, and the data were analyzed.

**8.2 Binding assay under pH 6.0 conditions**

The binding assay under pH 6.0 conditions was performed identically to the binding assay under pH 7.0 conditions, with pH 7.0 PBST being replaced by pH 6.0 PBST.

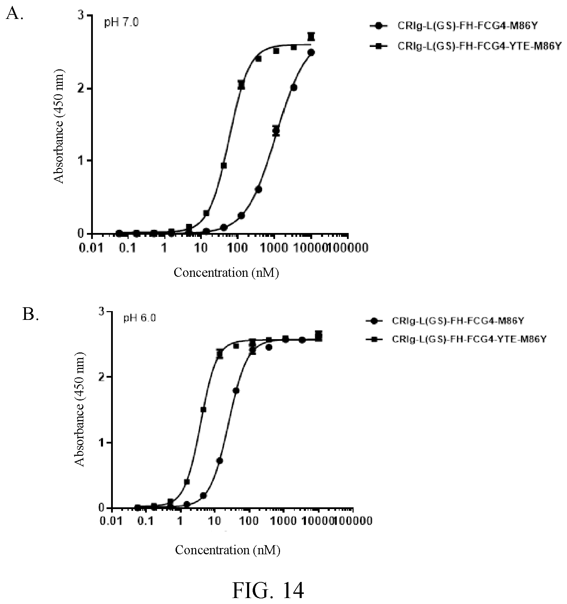

The experimental results are shown in Figure 14 and Table 14. The addition of the YTE mutation to the Fc region of CRIg-L(GS)-FH-FCG4-M86Y significantly enhanced its affinity for FcRn, with stronger affinity observed under pH 6.0 conditions. Therefore, theoretically, CRIg-L(GS)-FH-FCG4-YTE-M86Y should have a longer half-life in vivo.

Table 14: $EC_{50}$ values for the binding of fusion proteins to the FcRn-His protein

| pH | Sample | Mean (nM) | SD |
|---|---|---|---|
| pH 7.0 | CRIg-L(GS)-FH-FCG4-M86Y | 1111.000 | 131.522 |
| | CRIg-L(GS)-FH-FCG4-YTE-M86Y | 57.915 | 0.474 |
| pH 6.0 | CRIg-L(GS)-FH-FCG4-M86Y | 24.795 | 0.587 |
| | CRIg-L(GS)-FH-FCG4-YTE-M86Y | 3.841 | 0.012 |

**Example 9 Therapeutic effect of the fusion protein CRIg-L(GS)-FH-FCG4-YTE-M86Y on a rat passive Heymann nephritis (PHN) model**

Antibodies bind to specific antigens on the podocyte foot processes of rat glomerular epithelial cells, forming

immune complexes, which further activate the complement system via the alternative and lectin pathways, ultimately leading to massive proteinuria in rats. In this example, a rat passive Heymann nephritis (PHN) model was established by injecting 1 ml of sheep anti-rat Fx1A (rat kidney proximal tubule brush border antigen, purchased from PROBETEX, Cat# PTX-002S) antiserum into the tail vein of SD male rats. Twenty-five SD rats were randomly divided into 5 groups: a blank control group, a PHN model group, and CRIg-L(GS)-FH-FCG4-YTE-M86Y treatment groups at doses of 5 mg/kg, 10 mg/kg, and 20 mg/kg. Administration was performed twice, on the day of modeling and on Day 4, via tail vein injection, with an administration volume of 5 mg/kg. On Day 7 after modeling, a metabolic cage was used to collect 24-hour urine from the rats. The urine volume was recorded, and the urine protein concentration was measured (using a urine protein quantitative test kit (CBB method) purchased from Nanjing Jiancheng, Cat# C035-2-1). The total 24-hour urine protein amount was calculated.

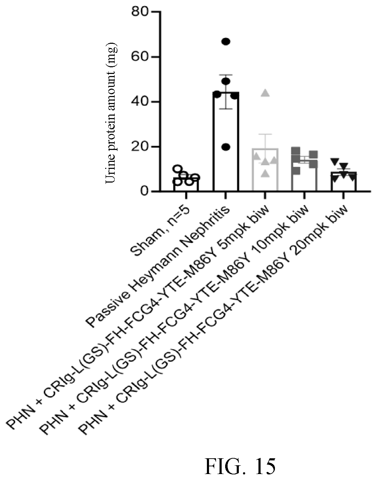

The results are shown in Figure 15. On Day 7 after modeling, the 24-hour urine protein amount in the PHN model group was significantly increased compared to the blank control group. The urine protein amount in the CRIg-L(GS)-FH-FCG4-YTE-M86Y treatment groups was significantly lower than that in the model group and showed a certain dose-dependent effect. This indicates that CRIg-L(GS)-FH-FCG4-YTE-M86Y can effectively bind to C3b in rats, inhibit complement system activation, and alleviate proteinuria caused by nephritis.

**Discussion:**

In the present invention, it was unexpectedly discovered that the M86Y mutation in the CRIg variant significantly enhanced the binding affinity of the fusion protein for human complement C3 compared to the wild-type protein. Furthermore, the fusion protein containing the M86Y mutation exhibited significantly improved performance in inhibiting red blood cell lysis induced by classical pathway activation, inhibiting alternative pathway activation, and inhibiting classical pathway activation, demonstrating unexpected technical effects.

**Sequences of the Invention**

SEQ ID NO. 1 CRIg

GGCCGGCCTATCCTGGAGGTGCCTGAGTCTGTGACCGGCCCTTGGAAGGGCGATGT
GAACCTGCCTTGCACCTACGATCCTCTGCAGGGCTACACCCAGGTTCTGGTGAAGTGGC
TGGTGCAGAGGGGCAGCGACCCCGTGACCATCTTTCTGCGCGACTCCTCCGGCGACCAC
ATCCAGCAGGCCAAGTACCAGGGCAGGCTGCACGTGTCCCACAAGGTGCCCGGCGACG
TGTCTCTGCAGCTGTCCACCCTGGAGATGGACGACCGCTCCCACTATACCTGCGAGGTG
ACCTGGCAGACTCCTGACGGCAACCAGGTGGTGAGAGATAAGATTACCGAG
CTGAGAGTGCAGAAG

SEQ ID NO. 2 CRIg

GRPILEVPESVTGPWKGDVNLPCTYDPLQGYTQVLVKWLVQRGSDPVTIFLRDSSGDH
IQQAKYQGRLHVSHKVPGDVSLQLSTLEMDDRSHYTCEVTWQTPDGNQVVRDKITELRVQ
K

SEQ ID NO. 3 Factor H

GAGGACTGTAATGAACTGCCTCCCAGAAGAAATACCGAAATCCTGACCGGCTCTTG
GTCTGATCAGACCTACCCAGAAGGCACACAGGCTATCTATAAATGTAGACCTGGGTATA
GAAGTCTGGGCAATGTGATCATGGTGTGTAGGAAGGGAGAGTGGGTGGCCCTGAATCC

TCTGAGAAAGTGCCAGAAGAGGCCTTGTGGCCATCCCGGCGATACCCCTTTTGGTACTT
TTACACTGACAGGCGGCAATGTGTTTGAGTATGGCGTGAAGGCCGTGTATACATGTAAT
GAGGGTTATCAGCTGCTGGGCGAGATTAATTATAGAGAGTGTGATACAGATGGCTGGA
CCAATGATATTCCTATTTGTGAAGTGGTGAAGTGTCTGCCTGTGACCGCCCCCGAGAAT
GGAAAGATCGTGAGCAGCGCCATGGAGCCCGATCGCGAGTACCATTTTGGCCAGGCTG
TGAGATTCGTGTGTAACAGCGGCTACAAGATCGAAGGCGACGAGGAGATGCACTGCTC
TGATGACGGCTTTTGGTCCAAGGAGAAGCCAAAGTGCGTGGAGATCTCCTGCAAGTCTC
CTGACGTGATCAACGGCAGCCCAATCTCTCAGAAGATCATCTACAAGGAGAATGAGAG
GTTCCAGTACAAGTGCAACATGGGCTACGAGTACTCCGAGAGAGGCGACGCTGTGTGC
ACCGAGTCTGGCTGGCGGCCTCTGCCAAGCTGCGAGGAGAAGTCCTGTGACAACCCTTA
TATCCCAAACGGCGACTACAGCCCTCTGAGAATCAAGCATAGGACAGGCGACGAGATC
ACCTACCAGTGTAGGAACGGCTTCTACCCCGCTACCAGAGGCAACACCGCCAAGTGTA
CCTCCACCGGCTGGATCCCTGCCCCCCGATGCACCCTGAAG

SEQ ID NO. 4 Factor H

EDCNELPPRRNTEILTGSWSDQTYPEGTQAIYKCRPGYRSLGNVIMVCRKGEWVALNP
LRKCQKRPCGHPGDTPFGTFTLTGGNVFEYGVKAVYTCNEGYQLLGEINYRECDTDGWTN
DIPICEVVKCLPVTAPENGKIVSSAMEPDREYHFGQAVRFVCNSGYKIEGDEEMHCSDDGF
WSKEKPKCVEISCKSPDVINGSPISQKIIYKENERFQYKCNMGYEYSERGDAVCTESGWRPL
PSCEEKSCDNPYIPNGDYSPLRIKHRTGDEITYQCRNGFYPATRGNTAKCTSTGWIPAPRCTL
K

SEQ ID NO. 5 IgG4-Fc

CCCCCATGCCCCTCCTGCCCTGCACCTGAGTTCCTGGGCGGCCCTTCCGTGTTCCTG
TTCCCCCCAAAGCCCAAGGATACCCTGATGATCAGCCGGACCCCTGAGGTGACCTGCGT
GGTGGTGGACGTGTCCCAGGAGGACCCCGAGGTGCAGTTCAACTGGTACGTGGACGGC
GTGGAGGTGCATAACGCCAAGACCAAGCCCAGAGAGGAGCAGTTTAACAGCACATACA
GAGTGGTGAGCGTGCTGACAGTGCTGCACCAGGACTGGCTGAATGGCAAGGAGTACAA
GTGCAAGGTGTCTAATAAGGGCCTGCCTAGCTCTATCGAAAAGACCATTTCTAAGGCTA
AGGGCCAGCCCAGAGAGCCACAGGTGTACACCCTGCCCCCTTCTCAGGAGGAGATGAC
CAAGAACCAGGTGTCTCTGACCTGCCTGGTGAAGGGCTTTTACCCTTCTGATATCGCCG
TGGAGTGGGAGAGCAACGGACAGCCTGAGAATAATTATAAGACCACTCCACCTGTGCT
GGATAGCGATGGCAGCTTCTTCCTGTACTCCAGACTGACTGTGGATAAGTCTAGGTGGC
AGGAGGGCAATGTGTTTTCCTGTAGTGTGATGCATGAGGCCCTGCATAATCATTATACC
CAGAAGTCTCTGTCTCTGTCCCTGGGCAAG

SEQ ID NO. 6 IgG4-Fc

PPCPSCPAPEFLGGPSVFLFPPKPKDTLMISRTPEVTCVVVDVSQEDPEVQFNWYVDGV
EVHNAKTKPREEQFNSTYRVVSVLTVLHQDWLNGKEYKCKVSNKGLPSSIEKTISKAKGQP
REPQVYTLPPSQEEMTKNQVSLTCLVKGFYPSDIAVEWESNGQPENNYKTTPPVLDSDGSF
FLYSRLTVDKSRWQEGNVFSCSVMHEALHNHYTQKSLSLSLGK

SEQ ID NO. 7 CRIg-L-FH-FCG4

GRPILEVPESVTGPWKGDVNLPCTYDPLQGYTQVLVKWLVQRGSDPVTIFLRDSSGDH
IQQAKYQGRLHVSHKVPGDVSLQLSTLEMDDRSHYTCEVTWQTPDGNQVVRDKITELRVQ
KSGGGGSGGGGSGGGGEDCNELPPRRNTEILTGSWSDQTYPEGTQAIYKCRPGYRSLGNVI
MVCRKGEWVALNPLRKCQKRPCGHPGDTPFGTFTLTGGNVFEYGVKAVYTCNEGYQLLG
EINYRECDTDGWTNDIPICEVVKCLPVTAPENGKIVSSAMEPDREYHFGQAVRFVCNSGYKI
EGDEEMHCSDDGFWSKEKPKCVEISCKSPDVINGSPISQKIIYKENERFQYKCNMGYEYSER
GDAVCTESGWRPLPSCEEKSCDNPYIPNGDYSPLRIKHRTGDEITYQCRNGFYPATRGNTAK
CTSTGWIPAPRCTLKPPCPPCPAPEFLGGPSVFLFPPKPKDTLMISRTPEVTCVVVDVSQEDP
EVQFNWYVDGVEVHNAKTKPREEQFNSTYRVVSVLTVLHQDWLNGKEYKCKVSNKGLPS
SIEKTISKAKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLGK

SEQ ID NO. 8 CRIg-L(GS)-FH-FCG4

GRPILEVPESVTGPWKGDVNLPCTYDPLQGYTQVLVKWLVQRGSDPVTIFLRDSSGDH
IQQAKYQGRLHVSHKVPGDVSLQLSTLEMDDRSHYTCEVTWQTPDGNQVVRDKITELRVQ
KGGGGSGGGGSGGGGSEDCNELPPRRNTEILTGSWSDQTYPEGTQAIYKCRPGYRSLGNVI
MVCRKGEWVALNPLRKCQKRPCGHPGDTPFGTFTLTGGNVFEYGVKAVYTCNEGYQLLG
EINYRECDTDGWTNDIPICEVVKCLPVTAPENGKIVSSAMEPDREYHFGQAVRFVCNSGYKI

EGDEEMHCSDDGFWSKEKPKCVEISCKSPDVINGSPISQKIIYKENERFQYKCNMGYEYSER
GDAVCTESGWRPLPSCEEKSCDNPYIPNGDYSPLRIKHRTGDEITYQCRNGFYPATRGNTAK
CTSTGWIPAPRCTLKPPCPPCPAPEFLGGPSVFLFPPKPKDTLMISRTPEVTCVVVDVSQEDP
EVQFNWYVDGVEVHNAKTKPREEQFNSTYRVVSVLTVLHQDWLNGKEYKCKVSNKGLPS
SIEKTISKAKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLGK

SEQ ID NO. 9 CRIg-L(GS)-FH-FCG4-Q64R

GRPILEVPESVTGPWKGDVNLPCTYDPLQGYTQVLVKWLVQRGSDPVTIFLRDSSGDH
IQQAKYRGRLHVSHKVPGDVSLQLSTLEMDDRSHYTCEVTWQTPDGNQVVRDKITELRVQ
KGGGGSGGGGSGGGGSEDCNELPPRRNTEILTGSWSDQTYPEGTQAIYKCRPGYRSLGNVI
MVCRKGEWVALNPLRKCQKRPCGHPGDTPFGTFTLTGGNVFEYGVKAVYTCNEGYQLLG
EINYRECDTDGWTNDIPICEVVKCLPVTAPENGKIVSSAMEPDREYHFGQAVRFVCNSGYKI
EGDEEMHCSDDGFWSKEKPKCVEISCKSPDVINGSPISQKIIYKENERFQYKCNMGYEYSER
GDAVCTESGWRPLPSCEEKSCDNPYIPNGDYSPLRIKHRTGDEITYQCRNGFYPATRGNTAK
CTSTGWIPAPRCTLKPPCPPCPAPEFLGGPSVFLFPPKPKDTLMISRTPEVTCVVVDVSQEDP
EVQFNWYVDGVEVHNAKTKPREEQFNSTYRVVSVLTVLHQDWLNGKEYKCKVSNKGLPS
SIEKTISKAKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLGK

SEQ ID NO. 10 CRIg-L(GS)-FH-FCG4-M86Y

GRPILEVPESVTGPWKGDVNLPCTYDPLQGYTQVLVKWLVQRGSDPVTIFLRDSSGDH
IQQAKYQGRLHVSHKVPGDVSLQLSTLEYDDRSHYTCEVTWQTPDGNQVVRDKITELRVQ
KGGGGSGGGGSGGGGSEDCNELPPRRNTEILTGSWSDQTYPEGTQAIYKCRPGYRSLGNVI
MVCRKGEWVALNPLRKCQKRPCGHPGDTPFGTFTLTGGNVFEYGVKAVYTCNEGYQLLG
EINYRECDTDGWTNDIPICEVVKCLPVTAPENGKIVSSAMEPDREYHFGQAVRFVCNSGYKI
EGDEEMHCSDDGFWSKEKPKCVEISCKSPDVINGSPISQKIIYKENERFQYKCNMGYEYSER
GDAVCTESGWRPLPSCEEKSCDNPYIPNGDYSPLRIKHRTGDEITYQCRNGFYPATRGNTAK
CTSTGWIPAPRCTLKPPCPPCPAPEFLGGPSVFLFPPKPKDTLMISRTPEVTCVVVDVSQEDP
EVQFNWYVDGVEVHNAKTKPREEQFNSTYRVVSVLTVLHQDWLNGKEYKCKVSNKGLPS
SIEKTISKAKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLGK

SEQ ID NO. 11 CRIg-L(GS)-FH-FCG4-Q64R-M86Y

GRPILEVPESVTGPWKGDVNLPCTYDPLQGYTQVLVKWLVQRGSDPVTIFLRDSSGDH IQQAKYRGRLHVSHKVPGDVSLQLSTLEYDDRSHYTCEVTWQTPDGNQVVRDKITELRVQ KGGGGSGGGGSGGGGSEDCNELPPRRNTEILTGSWSDQTYPEGTQAIYKCRPGYRSLGNVI MVCRKGEWVALNPLRKCQKRPCGHPGDTPFGTFTLTGGNVFEYGVKAVYTCNEGYQLLG EINYRECDTDGWTNDIPICEVVKCLPVTAPENGKIVSSAMEPDREYHFGQAVRFVCNSGYKI EGDEEMHCSDDGFWSKEKPKCVEISCKSPDVINGSPISQKIIYKENERFQYKCNMGYEYSER GDAVCTESGWRPLPSCEEKSCDNPYIPNGDYSPLRIKHRTGDEITYQCRNGFYPATRGNTAK CTSTGWIPAPRCTLKPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKEYKCKVSNKGLPS SIEKTISKAKGQPREPQVYTLPPSQEEMTKNQVSLTCLVKGFYPSDIAVEWESNGQPENNYK TTPPVLDSDGSFFLYSRLTVDKSRWQEGNVFSCSVMHEALHNHYTQKSLSLSLGK

SEQ ID NO. 12 CRIg-FH-FCG4-Q64R

GRPILEVPESVTGPWKGDVNLPCTYDPLQGYTQVLVKWLVQRGSDPVTIFLRDSSGDH IQQAKYRGRLHVSHKVPGDVSLQLSTLEMDDRSHYTCEVTWQTPDGNQVVRDKITELRVQ KEDCNELPPRRNTEILTGSWSDQTYPEGTQAIYKCRPGYRSLGNVIMVCRKGEWVALNPLR KCQKRPCGHPGDTPFGTFTLTGGNVFEYGVKAVYTCNEGYQLLGEINYRECDTDGWTNDI PICEVVKCLPVTAPENGKIVSSAMEPDREYHFGQAVRFVCNSGYKIEGDEEMHCSDDGFWS KEKPKCVEISCKSPDVINGSPISQKIIYKENERFQYKCNMGYEYSERGDAVCTESGWRPLPS CEEKSCDNPYIPNGDYSPLRIKHRTGDEITYQCRNGFYPATRGNTAKCTSTGWIPAPRCTLK PPCPPCPAPEFLGGPSVFLFPPKPKDTLMISRTPEVTCVVVDVSQEDPEVQFNWYVDGVEVH NAKTKPREEQFNSTYRVVSVLTVLHQDWLNGKEYKCKVSNKGLPSSIEKTISKAKGQPREP QVYTLPPSQEEMTKNQVSLTCLVKGFYPSDIAVEWESNGQPENNYKTTPPVLDSDGSFFLY SRLTVDKSRWQEGNVFSCSVMHEALHNHYTQKSLSLSLGK

SEQ ID NO. 13 CRIg -FH-FCG4-M86Y

GRPILEVPESVTGPWKGDVNLPCTYDPLQGYTQVLVKWLVQRGSDPVTIFLRDSSGDH

IQQAKYQGRLHVSHKVPGDVSLQLSTLEYDDRSHYTCEVTWQTPDGNQVVRDKITELRVQ KEDCNELPPRRNTEILTGSWSDQTYPEGTQAIYKCRPGYRSLGNVIMVCRKGEWVALNPLR KCQKRPCGHPGDTPFGTFTLTGGNVFEYGVKAVYTCNEGYQLLGEINYRECDTDGWTNDI PICEVVKCLPVTAPENGKIVSSAMEPDREYHFGQAVRFVCNSGYKIEGDEEMHCSDDGFWS KEKPKCVEISCKSPDVINGSPISQKIIYKENERFQYKCNMGYEYSERGDAVCTESGWRPLPS CEEKSCDNPYIPNGDYSPLRIKHRTGDEITYQCRNGFYPATRGNTAKCTSTGWIPAPRCTLK PPCPPCPAPEFLGGPSVFLFPPKPKDTLMISRTPEVTCVVVDVSQEDPEVQFNWYVDGVEVH NAKTKPREEQFNSTYRVVSVLTVLHQDWLNGKEYKCKVSNKGLPSSIEKTISKAKGQPREP QVYTLPPSQEEMTKNQVSLTCLVKGFYPSDIAVEWESNGQPENNYKTTPPVLDSDGSFFLY SRLTVDKSRWQEGNVFSCSVMHEALHNHYTQKSLSLSLGK

SEQ ID NO. 14 CRIg-FH-FCG4-Q64R-M86Y

GRPILEVPESVTGPWKGDVNLPCTYDPLQGYTQVLVKWLVQRGSDPVTIFLRDSSGDH IQQAKYRGRLHVSHKVPGDVSLQLSTLEYDDRSHYTCEVTWQTPDGNQVVRDKITELRVQ KEDCNELPPRRNTEILTGSWSDQTYPEGTQAIYKCRPGYRSLGNVIMVCRKGEWVALNPLR KCQKRPCGHPGDTPFGTFTLTGGNVFEYGVKAVYTCNEGYQLLGEINYRECDTDGWTNDI PICEVVKCLPVTAPENGKIVSSAMEPDREYHFGQAVRFVCNSGYKIEGDEEMHCSDDGFWS KEKPKCVEISCKSPDVINGSPISQKIIYKENERFQYKCNMGYEYSERGDAVCTESGWRPLPS CEEKSCDNPYIPNGDYSPLRIKHRTGDEITYQCRNGFYPATRGNTAKCTSTGWIPAPRCTLK PPCPPCPAPEFLGGPSVFLFPPKPKDTLMISRTPEVTCVVVDVSQEDPEVQFNWYVDGVEVH NAKTKPREEQFNSTYRVVSVLTVLHQDWLNGKEYKCKVSNKGLPSSIEKTISKAKGQPREP QVYTLPPSQEEMTKNQVSLTCLVKGFYPSDIAVEWESNGQPENNYKTTPPVLDSDGSFFLY SRLTVDKSRWQEGNVFSCSVMHEALHNHYTQKSLSLSLGK

SEQ ID NO. 15 CRIg-L(GS)-FH-FCG4-YTE -M86Y

GRPILEVPESVTGPWKGDVNLPCTYDPLQGYTQVLVKWLVQRGSDPVTIFLRDSSGDH IQQAKYQGRLHVSHKVPGDVSLQLSTLEYDDRSHYTCEVTWQTPDGNQVVRDKITELRVQ KGGGGSGGGGSGGGGSEDCNELPPRRNTEILTGSWSDQTYPEGTQAIYKCRPGYRSLGNVI MVCRKGEWVALNPLRKCQKRPCGHPGDTPFGTFTLTGGNVFEYGVKAVYTCNEGYQLLG EINYRECDTDGWTNDIPICEVVKCLPVTAPENGKIVSSAMEPDREYHFGQAVRFVCNSGYKI EGDEEMHCSDDGFWSKEKPKCVEISCKSPDVINGSPISQKIIYKENERFQYKCNMGYEYSER GDAVCTESGWRPLPSCEEKSCDNPYIPNGDYSPLRIKHRTGDEITYQCRNGFYPATRGNTAK CTSTGWIPAPRCTLKPPCPPCPAPEFLGGPSVFLFPPKPKDTLYITREPEVTCVVVDVSQEDP EVQFNWYVDGVEVHNAKTKPREEQFNSTYRVVSVLTVLHQDWLNGKEYKCKVSNKGLPS SIEKTISKAKGQPREPQVYTLPPSQEEMTKNQVSLTCLVKGFYPSDIAVEWESNGQPENNYK TTPPVLDSDGSFFLYSRLTVDKSRWQEGNVFSCSVMHEALHNHYTQKSLSLSLGK

All publications mentioned in the present invention are incorporated in the present application by reference to the same extent as if each individual publication was individually indicated to be incorporated by reference. Furthermore, it should be understood that, after reading the above teachings of the present invention, those skilled in the art can make various changes or modifications to the present invention, and these equivalent forms shall also fall within the scope defined by the claims attached to the present application.

## Claims

1. A C3b-targeting bifunctional fusion protein, **characterized by** comprising:

   a) a CRIg domain variant, wherein said variant comprises a mutation selected from the group consisting of: M86Y; or a combination of Q64R and M86Y, relative to SEQ ID NO: 2; and
   b) an FH domain.

2. The bifunctional fusion protein according to claim 1, **characterized in that** said C3b-targeting bifunctional fusion protein further comprises:

   c) a linker linking a) and b); and/or
   d) an Fc domain variant.

3. The bifunctional fusion protein according to claim 1, **characterized in that** said bifunctional fusion protein comprises a first polypeptide chain and a second polypeptide chain, wherein said first polypeptide chain comprises said CRIg domain variant, a first FH domain, and a first Fc domain variant; and said second polypeptide chain comprises said CRIg domain variant, a second FH domain, and a second Fc domain variant,
   wherein said first Fc domain variant and said second Fc domain variant are capable of interacting with each other to form a dimer.

4. The bifunctional fusion protein according to claim 2, **characterized in that** said Fc domain variant is an IgG4-Fc; preferably, it comprises an amino acid mutation S228P at position 228 of the human IgG4 constant region Fc sequence, and/or comprises a mutation selected from the group consisting of: M252Y, S254T, and T256E.

5. The bifunctional fusion protein according to claim 3, **characterized in that** the first polypeptide chain and the second polypeptide chain comprise an amino acid sequence as shown in any one of SEQ ID NOs: 7-15.

6. A nucleic acid molecule, **characterized in that** it encodes the bifunctional fusion protein according to any one of claims 1-5.

7. An expression vector, **characterized by** comprising the nucleic acid molecule according to claim 6.

8. A host cell, **characterized by** comprising the expression vector according to claim 7 or expresses the bifunctional fusion protein according to any one of claims 1-5.

9. A method for preparing the bifunctional fusion protein according to claim 1, **characterized by** comprising the steps of: synthesizing the bifunctional fusion protein according to any one of claims 1-5, and/or culturing the host cell according to claim 9 under conditions suitable for expressing the bifunctional fusion protein according to any one of claims 1-5.

10. A pharmaceutical composition, **characterized by** comprising the bifunctional fusion protein according to claim 1 and optionally a pharmaceutically acceptable carrier.

11. Use of the bifunctional fusion protein according to any one of claims 1-5 or the pharmaceutical composition according to claim 10 in the manufacture of a medicament, **characterized in that** said medicament is for preventing or treating a disease associated with complement activation.

12. Use of the bifunctional fusion protein according to any one of claims 1-5 or the pharmaceutical composition according to claim 10 in the manufacture of a medicament, **characterized in that** said medicament is for preventing or treating a disease selected from the group consisting of: paroxysmal nocturnal hemoglobinuria (PNH), atypical hemolytic uremic syndrome (aHUS), generalized myasthenia gravis (gMG), neuromyelitis opticas, age-related macular degeneration (AMD), geographic atrophy (GA), autoimmune hemolytic anemia, autoimmune thrombocytopenia, aplastic anemia, systemic lupus erythematosus, rheumatoid arthritis, ankylosing spondylitis, atherosclerosis, Parkinson's disease, Alzheimer's disease (senile dementia), asthma, allergy, psoriasis, IgA nephropathy, glomerulonephritis, lupus nephritis, multiple sclerosis, acute respiratory distress syndrome, and inflammatory diseases of periodontal tissues (including gingivitis and periodontitis).

13. A method for preventing or treating a disease associated with complement activation, **characterized by** comprising administering a pharmaceutically effective amount of the fusion protein according to any one of claims 1-5 or the pharmaceutical composition according to claim 10 to a subject in need thereof.

14. The method according to claim 13, **characterized in that** said disease associated with complement activation is selected from the group consisting of: paroxysmal nocturnal hemoglobinuria (PNH), atypical hemolytic uremic syndrome (aHUS), generalized myasthenia gravis (gMG), neuromyelitis optica, age-related macular degeneration (AMD), geographic atrophy (GA), autoimmune hemolytic anemia, autoimmune thrombocytopenia, aplastic anemia, systemic lupus erythematosus, rheumatoid arthritis, ankylosing spondylitis, atherosclerosis, Parkinson's disease, Alzheimer's disease (senile dementia), asthma, allergy, psoriasis, IgA nephropathy, glomerulonephritis, lupus nephritis, multiple sclerosis, acute respiratory distress syndrome, and inflammatory diseases of periodontal tissues (including gingivitis and periodontitis).

2.5
2.0
1.5
1.0
0.5
0.0
-0.5
OD450nm
0.1
1
10
100
1000
Conc.(nM)
CRIg-FCG4
FH-FCG4
FH-L(GS)-CRIg-FCG4
CRIg-FH-FCG4
CRIg-L-FH-FCG4
CRIg-L(GS)-FH-FCG4

FIG. 1

2.5
2.0
1.5
1.0
0.5
0.0
Absorbance (450 nm)
0.01
0.1
1
10
100
Concentration (nM)
CRIg-FH-FCG4
CRIg-FH-FCG4-M86Y
CRIg-FH-FCG4-Q64R
CRIg-FH-FCG4-Q64R-M86Y

FIG. 2

Absorbance (450 nm)
Concentration (nM)
3
2
1
0
-1
0.01
0.1
1
10
100
1000
CRIg-FH-FCG4
CRIg-L(GS)-FH-FCG4
CRIg-L(GS)-FH-FCG4-Q64R
CRIg-L(GS)-FH-FCG4-M86Y
CRIg-L(GS)-FH-FCG4-Q64R-M86Y

FIG. 3

Alternative Pathway
Red blood cell lysis rate (%)
Concentration (nM)
150
100
50
0
-50
0.1
1
10
100
1000
10000
CRIg-FH-FCG4
CRIg-L(GS)-FH-FCG4
CRIg-L(GS)-FH-FCG4-M86Y
CRIg-L(GS)-FH-FCG4-Q64R-M86Y

FIG. 4

Classical Pathway
Red blood cell lysis rate (%)
Concentration (nM)
150
100
50
0
0.001 0.01 0.1 1 10 100 10001000000000
CRIg-FH-FCG4
CRIg-L(GS)-FH-FCG4
CRIg-L(GS)-FH-FCG4-M86Y
CRIg-L(GS)-FH-FCG4-Q64R-M86Y

FIG. 5

Alternative Pathway
150
100
50
0
-50
Inhibition rate (%)
0.1
1
10
Concentration (nM)
CRIg-L(GS)-FH-FCG4
CRIg-L(GS)-FH-FCG4-M86Y
CRIg-L(GS)-FH-FCG4-Q64R-M86Y

FIG. 6

Classical Pathway
150
100
50
0
Inhibition rate (%)
0.001
0.01
0.1
1
10
100
1000
10000
Concentration (nM)
CRIg-L(GS)-FH-FCG4
CRIg-L(GS)-FH-FCG4-M86Y
CRIg-L(GS)-FH-FCG4-Q64R-M86Y

FIG. 7

Lectin Pathway
150
120
90
60
30
Inhibition rate (%)
0.01
0.1
1
10
100
1000
10000
Concentration (nM)
CRIg-FH-FCG4
CRIg-L(GS)-FH-FCG4
CRIg-L(GS)-FH-FCG4-M86Y

FIG. 8

Alternative Pathway
150
120
90
60
30
0.01
0.1
1
10
100
Inhibition rate (%)
Concentration (nM)
CRIg-FH-FCG4
CRIg-L(GS)-FH-FCG4
CRIg-L(GS)-FH-FCG4-M86Y

FIG. 9

Classical Pathway
150
100
50
0
0.0001
0.001
0.01
0.1
1
10
100
1000
10000
Inhibition rate (%)
Concentration (nM)
CRIg-FH-FCG4
CRIg-L(GS)-FH-FCG4
CRIg-L(GS)-FH-FCG4-M86Y

FIG. 10

3
2
1
0
-1
0.001
0.01
0.1
1
10
Absorbance (450 nm)
Concentration (nM)
biotin-CRIg-L(GS)-FH-FCG4-M86Y
biotin-CRIg-L(GS)-FH-FCG4-YTE-M86Y

FIG. 11

Alternative Pathway
150
100
50
0
Red blood cell lysis rate (%)
0.0001 0.001 0.01 0.1 1 10 100 1000 10000
Concentration (nM)
CRIg-L(GS)-FH-FCG4-M86Y
CRIg-L(GS)-FH-FCG4-YTE-M86Y

FIG. 12

Classical Pathway
150
100
50
0
Red blood cell lysis rate (%)
0.001 0.01 0.1 1 10 100 1000 10000 100000
Concentration (nM)
CRIg-L(GS)-FH-FCG4-M86Y
CRIg-L(GS)-FH-FCG4-YTE-M86Y

FIG. 13

A.
pH 7.0
CRIg-L(GS)-FH-FCG4-M86Y
CRIg-L(GS)-FH-FCG4-YTE-M86Y
Absorbance (450 nm)
Concentration (nM)
0
1
2
3
0.01
0.1
1
10
100
1000
10000
100000

B.
pH 6.0
CRIg-L(GS)-FH-FCG4-M86Y
CRIg-L(GS)-FH-FCG4-YTE-M86Y
Absorbance (450 nm)
Concentration (nM)
0
1
2
3
0.01
0.1
1
10
100
1000
10000
100000

FIG. 14

80
60
40
20
0
Urine protein amount (mg)
Sham, n=5
Passive Heymann Nephritis
PHN + CRIg-L(GS)-FH-FCG4-YTE-M86Y 5mpk biw
PHN + CRIg-L(GS)-FH-FCG4-YTE-M86Y 10mpk biw
PHN + CRIg-L(GS)-FH-FCG4-YTE-M86Y 20mpk biw


FIG. 15

## INTERNATIONAL SEARCH REPORT

International application No.
**PCT/CN2024/131610**

**A. CLASSIFICATION OF SUBJECT MATTER**

C07K19/00(2006.01)i；C12N15/62(2006.01)i

According to International Patent Classification (IPC) or to both national classification and IPC

**B. FIELDS SEARCHED**

Minimum documentation searched (classification system followed by classification symbols)

IPC：C07K；C12N

Documentation searched other than minimum documentation to the extent that such documents are included in the fields searched

Electronic data base consulted during the international search (name of data base and, where practicable, search terms used)

CNKI, CNTXT, DWPI, ENTXT, ELSEVIER, ISI Web of Knowledge, 中国专利生物序列检索系统, China Patent Biological Sequence Search System, Genbank, EMBL, STN, PubMed, C3b, 双功能融合蛋白, 补体系统, CRIg, Factor H, FH, M86Y, Q64R, Fc, 连接子, 二聚体, 相关序列, related sequences, Bifunctional Fusion Proteins, Complement System, Linkers, Dimers

**C. DOCUMENTS CONSIDERED TO BE RELEVANT**

| Category* | Citation of document, with indication, where appropriate, of the relevant passages | Relevant to claim No. |
|---|---|---|
| Y | CN 115515985 A (SHANGHAI COMGEN PHARMACEUTICAL CO., LTD.) 23 December 2022 (2022-12-23)<br>description, paragraphs 9 and 115-135, and sequence listing, and claims 1-16 and 34-41 | 1-14 |
| Y | CN 102015763 A (GENENTECH, INC. et al.) 13 April 2011 (2011-04-13)<br>description, paragraph 20, and claims 1-16 | 1-14 |
| A | SG 165048 A1 (GENENTECH, INC.) 28 October 2010 (2010-10-28)<br>entire document | 1-14 |
| A | CN 108699121 A (GREENOVATION BIOTECH GMBH) 23 October 2018 (2018-10-23)<br>entire document | 1-14 |
| A | CN 104231085 A (FUDAN UNIVERSITY SHANGHAI CANCER CENTER) 24 December 2014 (2014-12-24)<br>entire document | 1-14 |
| A | CN 113583107 A (FUDAN UNIVERSITY et al.) 02 November 2021 (2021-11-02)<br>entire document | 1-14 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **23 January 2025** | **06 February 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/131610**

**C. DOCUMENTS CONSIDERED TO BE RELEVANT**

| Category* | Citation of document, with indication, where appropriate, of the relevant passages | Relevant to claim No. |
|---|---|---|
| A | CN 114805572 A (SHANGHAI JEMINCARE PHARMACEUTICALS CO., LTD. et al.) 29 July 2022 (2022-07-29) entire document | 1-14 |

Form PCT/ISA/210 (second sheet) (July 2022)

**INTERNATIONAL SEARCH REPORT**

International application No.
**PCT/CN2024/131610**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/131610**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13-14**
because they relate to subject matter not required to be searched by this Authority, namely:

Claims 13-14 relate to a method for treating a disease, which falls within subject matter for which a search is not performed as defined in PCT Rule 39.1(iv). In the present report, a search is performed on the basis of "a pharmaceutical use of the related substance".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/131610**

| Patent document cited in search report | Publication date (day/month/year) | Patent family member(s) | Publication date (day/month/year) |
|---|---|---|---|
| CN 115515985 A | 23 December 2022 | US 2023348554 A1 | 02 November 2023 |
| | | CA 3178088 A1 | 18 November 2021 |
| | | WO 2021228052 A1 | 18 November 2021 |
| | | EP 4151659 A1 | 22 March 2023 |
| | | EP 4151659 A4 | 24 July 2024 |
| | | JP 2023526218 A | 21 June 2023 |
| CN 102015763 A | 13 April 2011 | AR 071635 A1 | 30 June 2010 |
| | | AU 2009244300 A1 | 12 November 2009 |
| | | AU 2009244300 A2 | 14 April 2011 |
| | | AU 2009244300 B2 | 17 April 2014 |
| | | SI 2280996 T1 | 30 December 2016 |
| | | MX 2010011372 A | 12 November 2010 |
| | | WO 2009137605 A2 | 12 November 2009 |
| | | WO 2009137605 A3 | 21 January 2010 |
| | | TW 200948377 A | 01 December 2009 |
| | | TWI 508973 B | 21 November 2015 |
| | | CL 2009001083 A1 | 07 January 2011 |
| | | CY 1118240 T1 | 28 June 2017 |
| | | CA 2720685 A1 | 12 November 2009 |
| | | CA 2720685 C | 18 April 2017 |
| | | KR 20170001711 A | 04 January 2017 |
| | | IL 249492 A0 | 28 February 2017 |
| | | US 2016008427 A1 | 14 January 2016 |
| | | JP 2011519950 A | 14 July 2011 |
| | | JP 5836800 B2 | 24 December 2015 |
| | | EP 2280996 A2 | 09 February 2011 |
| | | EP 2280996 B1 | 07 September 2016 |
| | | SG 190596 A1 | 28 June 2013 |
| | | PE 20141549 A1 | 25 October 2014 |
| | | ES 2605471 T3 | 14 March 2017 |
| | | IL 208379 A0 | 30 December 2010 |
| | | IL 208379 A | 29 December 2016 |
| | | US 2010150908 A1 | 17 June 2010 |
| | | US 9234024 B2 | 12 January 2016 |
| | | HUE 030092 T2 | 29 May 2017 |
| | | HRP 20161580 T1 | 30 December 2016 |
| | | BRPI 0912003 A2 | 12 July 2016 |
| | | EP 3141560 A2 | 15 March 2017 |
| | | EP 3141560 A3 | 24 May 2017 |
| | | PE 20091923 A1 | 19 December 2009 |
| | | KR 20110020798 A | 03 March 2011 |
| | | KR 101690590 B1 | 28 December 2016 |
| | | ZA 201006937 B | 25 January 2012 |
| | | DK 2280996 T3 | 19 December 2016 |
| | | HK 1210184 A1 | 15 April 2016 |
| | | LT 2280996 T | 12 December 2016 |
| | | PL 2280996 T3 | 31 July 2017 |
| | | PT 2280996 T | 06 December 2016 |
| | | RU 2010149804 A | 20 June 2012 |
| | | RU 2553517 C2 | 20 June 2015 |

Form PCT/ISA/210 (patent family annex) (July 2022)

**INTERNATIONAL SEARCH REPORT**
**Information on patent family members**

International application No.
**PCT/CN2024/131610**

| Patent document cited in search report | Publication date (day/month/year) | Patent family member(s) | Publication date (day/month/year) |
|---|---|---|---|
| | | JP 2015145369 A | 13 August 2015 |
| | | RU 2015117054 A | 20 October 2015 |
| SG 165048 A1 | 28 October 2010 | None | |
| CN 108699121 A | 23 October 2018 | PL 3394089 T3 | 17 January 2022 |
| | | DK 3394089 T3 | 01 November 2021 |
| | | LT 3394089 T | 25 November 2021 |
| | | MX 2018007392 A | 15 August 2018 |
| | | JP 2019508022 A | 28 March 2019 |
| | | JP 6771568 B2 | 21 October 2020 |
| | | SI 3394089 T1 | 31 December 2021 |
| | | KR 20180091097 A | 14 August 2018 |
| | | US 2020277347 A1 | 03 September 2020 |
| | | US 11591378 B2 | 28 February 2023 |
| | | CA 3009393 A1 | 29 June 2017 |
| | | CA 3009393 C | 22 August 2023 |
| | | BR 112018012844 A2 | 04 December 2018 |
| | | JP 2020167999 A | 15 October 2020 |
| | | AU 2016375183 A1 | 19 July 2018 |
| | | AU 2016375183 B2 | 21 January 2021 |
| | | HRP 20211608 T1 | 21 January 2022 |
| | | PT 3394089 T | 03 November 2021 |
| | | US 2019300589 A1 | 03 October 2019 |
| | | US 10640540 B2 | 05 May 2020 |
| | | HUE 056019 T2 | 28 January 2022 |
| | | IL 260182 A | 31 July 2018 |
| | | IL 260182 B1 | 01 August 2023 |
| | | IL 260182 B2 | 01 December 2023 |
| | | ES 2895256 T3 | 18 February 2022 |
| | | WO 2017109208 A1 | 29 June 2017 |
| | | EP 3394089 A1 | 31 October 2018 |
| | | EP 3394089 B1 | 28 July 2021 |
| CN 104231085 A | 24 December 2014 | JP 2016535062 A | 10 November 2016 |
| | | US 2016326231 A1 | 10 November 2016 |
| | | US 9862757 B2 | 09 January 2018 |
| | | AU 2014317705 A1 | 14 April 2016 |
| | | AU 2014317705 B2 | 08 June 2017 |
| | | WO 2015032167 A1 | 12 March 2015 |
| CN 113583107 A | 02 November 2021 | None | |
| CN 114805572 A | 29 July 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Computational Molecular Biology. Oxford University Press, 1988
- Biocomputing: Informatics and Genome Projects. Academic Press, 1993
- Computer Analysis of Sequence Data, Part I. Humana Press, 1994
- Sequence Analysis in Molecular Biology. Academic Press, 1987
- Sequence Analysis Primer. M Stockton Press, 1991
- **CARILLO, H.** ; **LIPMAN, D.** *SIAM J. Applied Math.*, 1988, vol. 48, 1073
- **ALTSCHUL, S. et al.** BLAST Manual. NCBI NLM NIH
- **SAMBROOK et al.** Molecular Cloning: A Laboratory Manual. Cold Spring Harbor Laboratory Press, 1989
- Current Protocols in Molecular Biology